# EUROPÄISCHE PATENTANMELDUNG

(11) **EP 3 967 260 A1**
(43) Veröffentlichungstag der Anmeldung: **16.03.2022**
(21) Anmeldenummer: 21186464.0
(22) Anmeldetag: 19.07.2021
(51) Int. Cl.: A61B 34/30, B25J 9/06, B81B 3/00, F03G 7/00

(54) **LICHT-AKTUIERBARES FLEXIBLES INSTRUMENT**

(30) Priorität: 10.09.2020 DE 102020123630
(71) Anmelder: Karlsruher Institut für Technologie, 76131 Karlsruhe (DE)
(72) Erfinder: Mathis-Ullrich, Franziska, 76227 Karlsruhe (DE); Marzi, Carl Christian, 76137 Karlsruhe (DE)

(57) **Zusammenfassung**

Eine Vorrichtung (100), umfassend: eine Grundstruktur (A, B, C, D, E, F) mit zumindest einem integrierten Lichtleiter (110) und/oder einer ansteuerbaren Lichtquelle (112), wobei diese zwei Elemente (114, 116) oder zwei Elemente (114, 116) und ein Gelenkelement (118), welches zumindest teilweise zwischen den Elementen (114, 116) angeordnet ist, umfasst, dadurch gekennzeichnet, dass zumindest eines der Elemente (114, 116) und/oder das Gelenkelement (118) zumindest teilweise aus zumindest einem Licht-aktuierbaren Material (120) gebildet ist oder zumindest teilweise ein solches aufweist und damit jeweils einen Aktuator (122) mit zumindest einer dominanten oder nutzbaren Wirkrichtung (124) bildet, und der Lichtleiter (110) und/oder die ansteuerbare Lichtquelle (112) derart eingerichtet und in oder an der Grundstruktur (A, B, C, D) angeordnet sind, dass damit Licht zum oder ans Licht-aktuierbare Material (120) leitbar ist.

## Beschreibung

Die Erfindung betrifft eine zumindest teilweise Licht-aktuierbare Vorrichtung, ein System basierend auf einer solchen Vorrichtung, ein Verfahren zum Betreiben einer solchen Vorrichtung oder eines solchen Systems sowie dessen Verwendung in der Medizintechnik oder als flexibles Instrument oder Kontinuumsroboter.

### Technisches Gebiet und Stand der Technik

Gegenüber der offenen Chirurgie sind die Minimal- oder nichtinvasiven Eingriffe (sogenannte Schlüssellochchirurgie) aufgrund zahlreicher Vorteile für den Patienten die bevorzugten Verfahren in der Chirurgie. Kleine Einschnitte oder instrumentelle Führung durch natürliche Körperöffnungen können Patiententrauma deutlich reduzieren und die Erholungszeit nach einem Eingriff verringern, was wiederum zu einer Reduktion der Gesundheitskosten führt [1]. Jedoch wird bei der Schlüssellochchirurgie bei Verwendung starrer Instrumente in engen Räumen und Gefäßen die Ergonomie und Fingerfertigkeit des Chirurgen eingeschränkt und führt damit zu ergonomisch schwieriger Handhabung. Um diese Herausforderungen zu überwinden, wurden zahlreiche chirurgischen Roboter und Assistenzgeräte für die minimal-invasive Chirurgie entwickelt. Medizinische Roboter sind entweder rein ferngesteuerte (telemanipulierte) Maschinen oder weisen einen gewissen Automatisierungsgrad auf und interagieren zur Unterstützung des Chirurgen bei medizinischen Eingriffen. Die Roboterchirurgie ermöglicht das Sammeln qualitativer Informationen über vielfältige Sensoren und gibt dem menschlichen Bediener ein Feedback. Das Robotergerät stellt eine gesteigerte Stabilität und Genauigkeit bereit und ist daher ideal bei sich wiederholenden oder heiklen chirurgischen Eingriffen, die eine hohe Positioniergenauigkeit benötigen. Viele Assistenzsysteme werden von einem menschlichen Chirurgen überwacht und ferngesteuert und können sich in beengten oder gefährlichen Umgebungen befinden, bspw. in einem MRT-Gerät (Magnetresonanztomographie) oder während einer Röntgenuntersuchung des Patienten. Nachgebende flexible Kontinuumsroboter weisen ein hohes Potenzial als Werkzeuge bzw. Instrumente für minimal- oder nicht-invasive Operationen in vielfältigen medizinischen Anwendungen auf, da sie aufgrund ihrer Weichheit eine intrinsische Sicherheit für Gewebe darstellen und so auch das Risiko von Gewebeschäden verringern können. Darüber hinaus können solch flexiblen Roboter engste Räume, wie z.B. Gefäße, durchqueren, Objekte in komplexen Umgebungen handhaben und sich an gekrümmte oder kurvenartige Pfade im Raum anpassen [2].

Typischerweise ermöglichen flexible Kontinuumsroboter eine Miniaturisierung des chirurgischen Werkzeugs bzw. Instruments und ermöglichen somit eine Erreichbarkeit und Handhabung, die über den Rahmen herkömmlicher medizinischer Roboter mit starrem Werkzeug bzw. Instrument hinausgeht. Für die Verwendung in medizinischen Anwendungen wurden mehrere intrinsische (d.h. Betätigung aus der Hauptstruktur heraus) und extrinsische (d.h. Kraftübertragung durch die Hauptstruktur) betätigte flexible Roboter vorgeschlagen. Die Krümmung des Sehnen- oder Spannglied-/Kabel-angetriebenen Kontinuumsroboter [3, 4] oder der Multi-Backbone-Strukturen [5, 6] wird durch koordinierte Verschiebung der Kabel an der Roboterbasis gesteuert. Konzentrische Rohrkontinuumsroboter (sogenannte Concentric-Tube Roboter) [7] werden durch axiale Drehung und relative Translation von zwei oder mehr vorgebogenen bzw. vorgekrümmten konzentrischen Rohren angetrieben, um die Form der gesamten Struktur zu ändern, und es wurde gezeigt, dass sie bei chirurgischen Eingriffen wie Biopsien [8] eingesetzt werden können. Es wurden Versteifungshüllen vorgeschlagen, um die Steifigkeit in bestimmten Konfigurationen eines rohrförmigen Roboters in Kombination mit anderen Aktuierungsstrategien, wie bspw. Sehnen- oder Spannglied-angetriebenen Kontinuumsrobotern [9], zu steuern. Magnetisch gesteuerte, flexible Werkzeuge bzw. Instrumente werden aus einem flexiblen Körper mit einer magnetischen Spitze hergestellt. Durch Anlegen externer Magnetfelder und Gradienten über elektromagnetische Manipulationssysteme [10, 11] oder Magnetresonanztomographie (MRT)-Maschinen [12] werden Kräfte und Drehmomente an der Werkzeugspitze bzw. Instrumentenspitze erzeugt, um flexible Körper zu bewegen. Intrinsisch aktuierte Kontinuumsroboter umfassen Strukturen mit eingebetteten Mikromotoren [13] oder bewegen sich unter Verwendung des Formgedächtniseffekts von Formgedächtnislegierungen [14]. Das pneumatische [15] und hydraulische Aktuieren [16] flexibler Werkzeuge bzw. Instrumente ermöglicht ein hohes Maß an Sicherheit und MRT-Kompatibilität, da keine magnetischen und elektrischen Komponenten in der flexiblen Struktur vorhanden sind.

Flüssigkristallelastomere (LCE) sind eine Art intelligentes reaktives Material, das unter dem Einfluss externer Reize eine große elastische Formänderung aufweist. Das Anlegen eines Stimulus wie eines elektrischen Feldes oder einer Änderung der Temperatur führt zu einer Änderung der Orientierungsordnung der im LCE [17] enthaltenen mesogenen Monomere und induziert somit eine mechanische Belastung bzw. Verzerrung des Materials. Die induzierte Dehnung verursacht eine große reversible Verformung des LCE. Neue Ergebnisse zeigen, dass durch Zugabe von photoisomerisierbaren Gruppen wie Azobenzol die Aktivierung des LCE durch Lichtbestrahlung ermöglicht wird [18] . Aufgrund des makroskopischen Verformungseffekts wurden kürzlich lichtreaktive LCEs als geeignete Aktuatoren für drahtlose weiche Geräte wie künstliche Muskeln, Motoren, nachgebende robotische Geher (z.B. durch künstliche Zilien) [19, 20], Mikroschwimmer [21, 22] sowie eine selbstregulierende Iris [23] vorgeschlagen.

Bei existierenden flexiblen Instrumenten oder Kontinuumsrobotern (z.B. (robotische) Manipulatoren in der Medizin) wird häufig nur die Spitze des Instruments angesteuert (z.B. Kabelzüge, magnetisch ... etc.) . Das führt dazu, dass zwar die Bewegung der Spitze, nicht aber des gesamten Instrumenten-Körpers gesteuert werden kann. Mit der vorliegenden Erfindung kann die Bewegung des Körpers segmentweise gesteuert werden. Zudem weist ein durch Licht aktuiertes Instrument die folgenden Vorteile auf: 1) es ist sauber, 2) es ist sicher (kein Strom erforderlich ... etc.), 3) es ist schnell, und 4) es ist miniaturisierbar. Außerdem lassen sich gewisse Lichtreaktive Materialien selektiv über die Wellenlänge oder ein Spektrum an Wellenlängen, Intensität und Polarisierung des einfallenden Lichts steuern.

Die flexiblen Körper der meisten anderen Lösungen für flexible Instrumente oder Kontinuumsroboter weisen keine Möglichkeit zur Steuerung bzw. der gezielten Veränderung an Steifigkeit entlang des gesamten Instruments auf. Viele Aktuierungsmethoden von flexiblen Instrumenten oder Kontinuumsrobotern nutzen entweder metallische Komponenten (bspw. nicht im MRT nutzbar) und weisen eine Gefahr der Undichtigkeit auf (z.B. hydraulisch oder pneumatische Aktuierung, Austritt von Luft oder Flüssigkeiten in den Körper) oder benötigen große und teure externe Aktuierungssysteme (z.B. magnetische Aktuierung). Die mechanische Ansteuerung von Kontinuumsrobotern ist häufig schwer modellierbar (aufgrund von z.B. Reibungsverlusten), während im Vergleich dazu Licht verlustarm übertragbar ist.

Der nächstliegende Stand der Technik wird z.B. durch eine Licht-aktuierbare robotische "Raupe" beschrieben. Ein LCE Film wird mit Licht beschienen, sodass eine Wellen-ähnliche Bewegung dadurch induziert wird, mit der sich die Struktur fortbewegt [19]. Eine andere Quelle zeigt einen weichen Roboter auf, der sich aufgrund der Reaktion des Materials bei einfallendem Licht fortbewegt [21].

Beide Systeme benötigen externe Lichtquellen und haben keine integrierten Lichtleiter oder integrierte Lichtquellen zur Aktuierung des Materials.

Sowohl Kontinuumsroboter als auch LCE Materialien oder Hydrogele sind Stand der Technik. Dennoch existieren bisher noch keine Kontinuumsroboter, welche über Lichteinfall aktuiert werden. Die bisherigen LCE aktuierten Systeme werden im Allgemeinen von außen mit Licht aktuiert und integrieren nicht Lichtleiter in der Struktur selbst.

Bisherige Lösungsansätze konnten jedoch nicht folgende Probleme überwinden:
- Die flexiblen Körper der meisten anderen Lösungen für Kontinuumsroboter weisen keine Möglichkeit zur Steuerung bzw. der gezielten Veränderung von Steifigkeit entlang des gesamten Instruments auf.
- Viele Aktuierungsmethoden von kommerziellen Kontinuumsrobotern nutzen entweder metallische Komponenten (d.h. sie sind damit nicht im MRT nutzbar), weisen Gefahr der Undichtigkeit auf (z.B. hydraulisch oder pneumatische Aktuierung, Austritt von Luft oder Flüssigkeiten in den Körper) oder benötigen große und teure externe Aktuierungssysteme (wie z.B. magnetische Aktuierung) .
- Die mechanische Ansteuerung von Kontinuumsrobotern ist häufig schwer modellierbar (aufgrund z.B. Reibungsverlusten), während im Vergleich dazu Licht verlustarm übertragbar ist.

### Aufgabe

Bereitstellen einer Vorrichtung und eines Verfahrens zum Betreiben einer solchen Vorrichtung, die eine Grundstruktur bilden welche eine Verwendung im medizinischen oder industriellen Bereich unter räumlichen eingeschränkten Bedingungen ermöglicht sowie zumindest eine licht-basierte Steuerung und zumindest einen lokal begrenzten Aktuierungs- und/oder Elastizitätsbereich derart aufweist, dass sich daraus Systeme bilden lassen, die wiederum als flexible Instrumente oder Kontinuumsroboter einsetzbar sind.

### Lösung

Diese zuvor genannte objektiv technische Aufgabe wird mit der hierin offenbarten Vorrichtung gemäß den Merkmalen des ersten, unabhängigen Anspruchs 1 gelöst. Hierauf bezogene Neben- bzw. Unteransprüche geben vorteilhafte Ausgestaltungen bzw. Ausführungsformen wider. Vorteilhafte Weiterbildungen, welche einzeln oder in beliebiger Kombination realisierbar sind, sind in den abhängigen Ansprüchen dargestellt.

Im Folgenden werden die Begriffe "aufweisen", "umfassen" oder "einschließen" oder beliebige grammatikalische Abweichungen davon in nicht-ausschließlicher Weise verwendet. Dementsprechend können sich diese Begriffe sowohl auf Situationen beziehen, in welchen, neben den durch diese Begriffe eingeführten Merkmalen, keine weiteren Merkmale vorhanden sind, oder auf Situationen, in welchen ein oder mehrere weitere Merkmale vorhanden sind. Beispielsweise kann sich der Ausdruck "A weist B auf", "A umfasst B" oder "A schließt B ein" sowohl auf die Situation beziehen, in welcher, abgesehen von B, kein weiteres Element in A vorhanden ist (d.h. auf eine Situation, in welcher A ausschließlich aus B besteht), als auch auf die Situation, in welcher, zusätzlich zu B, ein oder mehrere weitere Elemente in A vorhanden sind, beispielsweise Element C, Elemente C und D oder sogar weitere Elemente.

Weiterhin wird darauf hingewiesen, dass die Begriffe "mindestens ein" und "ein oder mehrere" sowie grammatikalische Abwandlungen dieser Begriffe, wenn diese in Zusammenhang mit einem oder mehreren Elementen oder Merkmalen verwendet werden und ausdrücken sollen, dass das Element oder Merkmal einfach oder mehrfach vorgesehen sein kann, in der Regel lediglich einmalig verwendet werden, beispielsweise bei der erstmaligen Einführung des Merkmals oder Elementes. Bei einer nachfolgenden erneuten Erwähnung des Merkmals oder Elementes wird der entsprechende Begriff "mindestens ein" oder "ein oder mehrere" in der Regel nicht mehr verwendet, ohne Einschränkung der Möglichkeit, dass das Merkmal oder Element einfach oder mehrfach vorgesehen sein kann.

Weiterhin werden im Folgenden die Begriffe "vorzugsweise", "insbesondere", "beispielsweise (bspw.)" oder ähnliche Begriffe in Verbindung mit optionalen Merkmalen verwendet, ohne dass alternative Ausführungsformen hierdurch beschränkt werden. So sind Merkmale, welche durch diese Begriffe eingeleitet werden, optionale Merkmale, und es ist nicht beabsichtigt, durch diese Merkmale den Schutzumfang der Ansprüche und insbesondere der unabhängigen Ansprüche einzuschränken. So kann die Erfindung, wie der Fachmann erkennen wird, auch unter Verwendung anderer Ausgestaltungen durchgeführt werden. In ähnlicher Weise werden Merkmale, welche durch "in einer Ausführungsform" oder durch "in einer weiteren Ausführungsform" eingeleitet werden, als optionale Merkmale verstanden, ohne dass hierdurch alternative Ausgestaltungen oder der Schutzumfang der unabhängigen Ansprüche eingeschränkt werden soll. Weiterhin sollen durch diese einleitenden Ausdrücke sämtliche Möglichkeiten, hierdurch eingeleitete Merkmale mit anderen Merkmalen zu kombinieren, seien es optionale oder nicht-optionale Merkmale, unangetastet bleiben.

### Allgemeine Beschreibung und Ausführungsformen

Licht-aktuierbare Materialien ermöglichen eine Aktuierung mittels Lichts anstatt von Elektrizität, Wärme, pH oder Ähnlichem. Eine Aktuierung mittels oder durch Licht ermöglicht bei visuellen oder Infrarot (IR) Wellenlängen keine schädigende Wechselwirkung mit biologischem Gewebe, wodurch diese sicher für den Einsatz im medizinischen Bereich sind. Zusätzlich ermöglicht Aktuierung durch Licht im UV-Spektrum die Aktuierung solcher Materialien. Durch Führung von UV Licht in Lichtleitern ist die Wirkung auf menschliches Gewebe reduziert und dieses kann sicher im menschlichen Körper angewendet werden. Darüber hinaus ermöglicht ein Verzicht auf metallischen Komponenten in einer erfindungsgemäßen Vorrichtung oder einem darauf basierenden System Ausführungsformen, welche MRT-kompatibel sind oder weniger Defekte (sogenannte Artefakte) in CT Bildern erzeugen, wodurch sich Störungen in bildgebenden Verfahren verringern lassen. Licht-aktuierbares Material ermöglicht eine Miniaturisierung von darauf basierenden Vorrichtungen, da dieses Material bereits ohne vorherige Mikro-Strukturierung bzw. Komposition von solchem Material und Formstücken (wie bspw. bei E-Motoren) als Aktuator wirkt, welche bisher bei herkömmlichen Materialien erforderlich war. Folglich können auf Licht-aktuierbarem Material basierende miniaturisierte Systeme für die Kontinuumsrobotik oder Medizintechnik gebildet werden.

Mittels integriertem Lichtleiter oder Lichtquelle ist es möglich einen lokal begrenzten Bereich als Freiheitsgrad (FG) selektiv anzusteuern. Hierdurch ist in Verbindung mit Licht-aktuierbaren Elementen eine Steuerung platzsparender gegenüber bspw. elektronischen Schaltungen realisierbar und eine solche Anordnung bietet eine gesteigerte Sicherheit durch Integration der Fasern innerhalb der Struktur. Darüber hinaus bietet eine solche Integration auch eine flexiblere (Energie-)Versorgung und Steuerleitung und verlustärmere Übertragung - auch an schwer erreichbare Stellen - mit sehr dünnem Durchmesser, was wiederum für die Miniaturisierung von Vorteil ist. D.h. eine erfindungsgemäße Vorrichtung kann durch die sehr lokal begrenzte Selektivität eine sehr hohe Integrationsdichte von FG erreichen bzw. Miniaturisierung ermöglichen. Dies ist insbesondere für die minimal invasive Chirurgie aufgrund guter Erreichbarkeit und Manipulierbarkeit vorteilhaft. Auch wird dadurch eine Erzeugung von Licht in großer Entfernung und Leitung zum Aktuator ermöglicht, wodurch platzsparende Ausführungen am Einsatzort realisierbar sind. Außerdem ist ein Transport bzw. Übertragung von Licht hin zum Aktuator ohne unerwünschte Effekte aus der Lichtquelle wie bspw. Wärme umsetzbar.

Eine integrierte Lichtquelle erlaubt eine vereinfachte elektronische Ansteuerung und individuelle Aktuierung jedes Gelenkes oder Elements, bspw. mittels LED mit wählbarer Wellenlänge und Intensität des Lichts. Sie sind optional zu einem Lichtleiter einsetzbar.

Eine flexible Grundstruktur mit lokal wählbarer Elastizität mit integrierter, selektiver Aktuierung ermöglicht eine hohe Erreichbarkeit und Manipulierbarkeit einer darauf basierenden Vorrichtung oder eines darauf aufgebauten Systems. Ein solcher Aufbau bietet eine verbesserte Effektivität der Wirkung gegenüber herkömmlichen Lösungen sowie eine geringere Dämpfung der Aktuierung. Die Nachgiebigkeit bei Krafteinwirkung von außen vermeidet dabei gefährliche Kräfte und Momente und damit Verletzung bei Einsatz im medizinischen Bereich. Aufgrund dieser strukturellen Merkmale ergibt sich eine Unterscheidung von hoher Elastizität bspw. an beweglichen Gelenken und hoher Steifigkeit bspw. zur Aufnahme oder Integration von Endeffektoren/Werkzeugen sowie Sensoren.

Die dominante Wirkrichtung ermöglicht die Verwendung einer erfindungsgemäßen Vorrichtung als kontrollierbarer Aktuator, erlaubt Ansteuerung und Nutzbarmachung der Bewegung.

Die wählbare Elastizität ermöglicht eine gesteigerte Entwurfsfreiheit zur Realisierung verschiedener Vorzugsrichtungen für verschiedene Aktuatoren. Hierdurch können verschiedene Aktuatoren für unterschiedliche Einsatzzwecke oder Gelenkpositionen gebildet werden, bspw. durch Verwendung von vernetztem Polydimethylsiloxan (PDMS) oder Silikonen verschiedener Shore-Härten (Shore-Härten von Elastomeren und gummiartige Polymeren definiert in DIN EN ISO 868 und DIN ISO 7619-1).

Die Elastizität ist bspw. wählbar durch:
1) Materialkomposition (z.B. PDMS oder Silikone unterschiedlicher Shore-Härten, Wahl des Materials von hartem bis sehr weichem Polymer alles möglich),
2) lokale Materialschwächung/strukturelle Schwächung (z.B. durch gewählte Einschnitte, Dicke des Materials, Porosität, etc.)

Eine Verwendung von Komposit in einer erfindungsgemäßen Vorrichtung oder einem darauf basierenden System ermöglicht die Kombination verschiedener Materialien, um dadurch ihre Materialeigenschaften (bspw. physikalisch) zu kombinieren. D.h. die Nutzung von Vorteilen der jeweiligen Eigenschaften beider Materialien, bspw. Biokompatibilität, Verarbeitbarkeit des Materials, Sensibilisierung auf andere Stimuli als Licht oder sensorische Eigenschaften. Darüber hinaus ergibt sich die Möglichkeit einer reversiblen Ansteuerung: bspw. als Bilayer oder Komposit, bei welchen unterschiedliche Reaktionen hervorgerufen werden können (z.B. Selektivität: Ein Material reagiert auf Licht der Wellenlänge λ₁ und das andere auf Wellenlänge λ₂, d.h. auf unterschiedliche Wellenlängen).

Sowohl Kontinuumsroboter als auch LCE Materialien und Hydrogele sind im Stand der Technik bekannt. Dennoch existieren keine Kontinuumsroboter, welche über Lichteinfall aktuierbar sind. Die bisherigen Licht-aktuierbare Systeme (vgl. oben Stand der Technik) werden im Allgemeine von außen mit Licht aktuiert und weisen keine integrierten Lichtleiter oder integrierte, ansteuerbare Lichtquellen in deren Struktur selbst auf.

Dadurch, dass eine erfindungsgemäße Vorrichtung oder System die Lichtleiter oder die ansteuerbare Lichtquelle bzw. eine Mehrzahl davon in das Instrument bzw. deren Grundstruktur selbst integrieren (und damit die LCEs oder Hydrogele nicht mit einer externen direkten Lichtquelle bestrahlen müssen), benötigt die Ansteuerung der Aktuatoren keine direkte Sichtverbindung. So kann eine solche Vorrichtung oder System innerhalb von undurchsichtigen Strukturen (z.B. Rohre/Rohrsysteme oder im Körper) über eine zu überbrückende Distanz angesteuert werden. Zudem ergibt sich eine hohe Selektivität der angesteuerten Elemente.

Eine erfindungsgemäße Vorrichtung umfasst bspw. zumindest folgende Komponenten: eine flexible Grundstruktur, aktuierte Gelenke und einen integrierten Lichtleiter oder eine integrierte, ansteuerbare Lichtquelle. D.h.:
1) Flexible Grundstruktur (mit angepassten Materialeigenschaften, bspw. PDMS oder Silikon), optional mit strukturellen Merkmalen (bspw. Einkerbungen zum einfacheren "Abknicken"). Eine solche Grundstruktur kann entweder passiv sein mit angebrachten aktuierten Gelenken oder direkt zumindest teilweise aus aktuierbarem Material gebildet sein oder ein solches zumindest teilweise aufweisen.
2) Aktuierte Gelenke aus Licht-reaktiven oder Licht-aktuierbaren Materialien (engl. "liquid crystral elastomers": LCE, u.a. sind hierfür auch Hydrogele geeignet), welche große elastische Formänderungen unter dem Einfluss von äußeren Reizen bzw. Anregungen, wie bspw. Licht, aufweisen.
3) In die Grundstruktur integrierte Lichtleiter oder integrierte, ansteuerbare Lichtquellen, die das Licht zu jedem aktuierbaren Gelenk führen bzw. darauf applizieren.

Die aktuierbaren Gelenke des Kontinuumsroboter umfassen bspw. zumindest teilweise LCE Materialien (oder Hydrogele) oder sind zumindest teilweise daraus gebildet und sind somit mittels Lichteinstrahlung aktuierbar. Licht-Aktuation birgt die Vorteile, dass es sowohl sauber, sicher, schnell, als auch modulierbar (d.h. dessen Wellenlänge, Polarisierung und/oder Intensität ist anpassbar) ist und damit viele positive Eigenschaften für die Medizintechnik hat. Eine erfindungsgemäße Vorrichtung oder System kann aber auch in anderen Anwendungen (bspw. für eine Inspektion in Rohren oder Rohrsystemen, wie z.B. in der Chemie- oder Lebensmittelindustrie etc.) genutzt werden. Für die Medizin birgt das System weiterhin den Vorteil, dass keine metallischen Bauteile genutzt werden müssen und ein solches Instrument (d.h. Vorrichtung oder System) somit in einem beliebigen MRT oder CT genutzt werden kann (bspw. bei medizinischer Bildgebung).

Eine erste Ausführungsform einer erfindungsgemäßen Vorrichtung umfasst: eine Grundstruktur mit zumindest einem integrierten Lichtleiter und/oder einer ansteuerbaren Lichtquelle, wobei diese zwei Elemente oder zwei Elemente und ein Gelenkelement, welches zumindest teilweise zwischen den Elementen angeordnet ist, umfasst, dadurch gekennzeichnet, dass zumindest eines der Elemente und/oder das Gelenkelement zumindest teilweise aus zumindest einem Licht-aktuierbaren Material gebildet ist oder zumindest teilweise ein solches aufweist und damit jeweils einen Aktuator mit zumindest einer dominanten oder nutzbaren Wirkrichtung bildet, und der Lichtleiter und/oder die ansteuerbare Lichtquelle derart eingerichtet und in oder an der Grundstruktur angeordnet sind, dass damit Licht zum oder ans Licht-aktuierbare Material leitbar ist.

Eine solche Vorrichtung ermöglicht das Aktuieren eines flexiblen Instruments, welches optional über viele Freiheitsgraden verfügt, für Medizin und Inspektion von bspw. sehr engen "Kanälen", d.h. räumlich eingeschränkt und das sowohl im technischen Sinne, als auch bzgl. Organen/Gefäßen. Darüber hinaus ist eine Ansteuerung eines solchen Instruments derart von außen, dass dessen Spitze und der Instrumentenkörper kontrolliert bewegt werden können. Solch ein Instrumente kann derart eingerichtet sein, dass es sich für Einsatz im menschlichen Körper eignet und "sauber", d.h. im klinischen Sinne, ist, so dass keine Flüssigkeiten hinein- noch heraustreten bzw. hohe elektrische Ströme und/oder Spannungen zur Ansteuerung eingesetzt werden müssen, etc. Ebenso kann solch ein Instrument miniaturisierbar sein und schnell aktuiert werden, da das aktuierende Licht entsprechend schnell auf das Licht-aktuierbare Material applizierte werden kann. Solch ein Instrument kann auch in Verbindung mit medizinischer Bildgebung (z.B. CT, MRT) genutzt werden, d.h. wenn also keine Metalle darin verbaut sind. Außerdem ist solch ein Instrument erweiterbar mit Sensorik und Aktorik für Diagnose und Behandlung innerhalb eines Körpers.

In einer weiteren Ausführungsform ist zumindest eines der Elemente derart gebildet, dass es eine zuvor wählbare Elastizität aufweist.

Aufgrund der wählbaren Elastizität wird eine Anpassung der Krümmung der Grundstruktur bei gleicher Stärke der Licht-Aktuierung ermöglicht, d.h. bspw. wenn die Grundstruktur eher steif sein soll wird ein kleiner Winkel gebildet oder bereitgestellt oder wenn die Grundstruktur eher flexibel bzw. weich sein soll, ein entsprechend größerer Winkel gebildet oder bereitgestellt. Folglich ergibt sich daraus der Vorteil, dass die gewünschte Anpassung der Krümmung dadurch ermöglicht wird und somit eine hohe Flexibilität der Gesamtstrukturbewegung bereitgestellt wird. Außerdem kann eine Anpassung der Instrument-Eigenschaften an äußere Bedingungen (wie z.B. den Einsatzort in einem Körper) dadurch bereitgestellt werden.

In einer weiteren Ausführungsform ist zumindest eine der mindestens einen Wirkrichtung auf zumindest das andere Element, eines der anderen Elemente, das Gelenkelement oder eines der Gelenkelemente gerichtet. Hierdurch wird eine relative Bewegung (Translation oder Rotation) zwischen zwei oder mehr (aktuierbaren) Elementen erzeugt und somit ist eine Aktuierung einzelner Elemente gegeneinander möglich, wodurch sich eine kinematische Kette bilden lässt. Vorteilhaft dabei ist die sich ergebende Erhöhung der Anzahl von Freiheitsgraden eines solchen Instrumentes. Dies ermöglicht eine Steuerung der gesamten Form des flexiblen Instruments (bspw. auch elementweise) bzw. Steuerung der variablen Steifigkeit des gesamten Instrumentenkörpers und erschließt die Möglichkeit zum Umgehen von Hindernissen entlang des Instrumenten-Pfades.

In einer weiteren Ausführungsform weist das Licht-aktuierbare Material zumindest ein Material aus der Gruppe von Licht-aktuierbaren Materialien auf oder ist zumindest teilweise daraus gebildet, wobei diese Gruppe Hydrogele und/oder Flüssigkristallpolymere (und Flüssigkristalline Elastomere) umfasst.

Hydrogele und Flüssigkristallpolymere sind die bekannten Materialien, welche sich unter Licht-Einwirkung deformieren bzw. ihre Form verändern können. Die Material-Eigenschaften können entweder chemisch (z.B. durch Verwendung entsprechender Vernetzer wie bspw. Azobenzen oder Azotolene) und/oder physisch (z.B. durch Integration von Fasern oder gewählte Ausrichtung der Flüssigkristallstruktur) verändert und demgemäß an die gewünschte Verwendung angepasst werden. Mit anderen Worten kann somit das aktuierbare Material entsprechend der Anwendung (bzgl. Wirkrichtung, Zugfestigkeit und Elastizitätsmodul) ausgewählt und angepasst werden, z.B. hinsichtlich der Verträglichkeit mit der Anwendungsumgebung, benötigte Stärke der Deformation und/oder der Aktuierungsenergie.

In einer weiteren Ausführungsform ist mindestens ein Aktuator derart als Komposit gebildet, wobei: dieses Komposit jeweils zumindest zwei verbundene Materialien umfasst, und zumindest eines der Materialien zumindest teilweise Licht-aktuierbares Material aufweist oder daraus gebildet ist.

Die Kombination von Materialien (Komposit) verbessert die Steuerbarkeit der Dynamik des Aktuators. z.B. können Materialien einander entgegenwirken oder auf unterschiedliche Stimuli (Wellenlänge, Lichtintensität etc.) reagieren, sodass kombinierte Bewegungen ermöglicht werden. Folglich sind damit kombinierte Bewegungen möglich, das Design des Aktuators kann auf den Bestimmungsort (z.B. zu behandelndes Organ, Form des Rohrsystems) und die benötigte Art der Bewegung ausgelegt werden.

In einer weiteren Ausführungsform bewegen sich die Materialien entsprechend der Wirkrichtung des jeweiligen Licht-aktuierbaren Materials entweder entgegengesetzt zueinander, oder zumindest eines der Materialien weist Licht-aktuierbares Material mit einer Wirkrichtung auf oder ist daraus gebildet und zumindest eines der anderen Materialien ist frei von Licht-aktuierbarem Material.

Wenn eines der Materialien in einer erfindungsgemäßen Ausführung aktuierbar ist und zumindest eines der anderen nicht, dann kann eine relative Bewegung zwischen diesen Materialien oder Bauteilen stattfinden. Zudem kann das nicht-aktuierbare Material zur Befestigung oder Fixierung anderer Elemente genutzt werden, da dann keine relative Bewegung generiert wird. Folglich wird damit die Möglichkeit zur unabhängigen Bewegung einzelner Materialien oder Bauteile bereitgestellt, d.h. eine bessere Steuerung einer solche Vorrichtung. Ebenso kann damit die Möglichkeit zur Fixierung oder Befestigung eines solchen Elements an andere, bzw. an einer Basis bereitgestellt werden.

In einer weiteren Ausführungsform weist mindestens ein Aktuator zumindest einen Einschnitt oder eine lokale Materialschwächung derart auf, dass dadurch im Bereich des Einschnitts oder der lokalen Materialschwächung eine erhöhte elastische Nachgiebigkeit gebildet ist.

Eine wählbare Elastizität an der lokalen Materialschwächung ermöglicht eine Anpassung der Krümmung bei gleicher Stärke der Aktuierung (d.h. Licht-aktuiert d.h. bspw., wenn eine solche Grundstruktur eher steif sein soll wird ein kleiner Winkel gebildet oder bereitgestellt oder wenn die Grundstruktur eher flexibel bzw. weich sein soll, ein entsprechend größerer Winkel gebildet oder bereitgestellt. Folglich ergibt sich daraus der Vorteil, dass die gewünschte Anpassung der Krümmung dadurch ermöglicht wird und somit eine hohe Flexibilität der Gesamtstrukturbewegung bereitgestellt wird. Außerdem kann eine Anpassung der Instrument-Eigenschaften an äußere Bedingungen (wie z.B. den Einsatzort in einem Körper) dadurch bereitgestellt werden.

In einer weiteren Ausführungsform weist mindestens ein Aktuator zumindest eine mit zumindest einem Licht-aktuierbaren Material gefüllten Einschnitt oder eine lokale Materialschwächung derart auf, dass damit beim Aktuieren eine Vorzugrichtung implementiert wird.

Durch eine lokale, strukturelle Anpassung des Aktuators kann eine bevorzugte Deformation ermöglicht werden, sodass selbst ein eigentlich homogen ausdehnbares/deformiertes Material eine bevorzugte Wirkrichtung erhält. Vorteilhaft ist, dass dadurch die Deformation über die Form des Aktuators bestimmt wird, nicht oder nicht nur über die Änderung der Materialeigenschaften. Anpassung an notwendige Bewegung werden somit ermöglicht (d.h. steifer oder weicher an einer zuvor bestimmbaren Stelle oder Bereich am jeweiligen Element beeinflusst den aktuierbaren Winkel)
Eine Ausführungsform eines erfindungsgemäßen Systems, umfasst zwei Vorrichtungen gemäß einer der vorhergehenden Ausführungsformen auf, wobei diese Vorrichtungen miteinander gekoppelt sind.

Eine solche Ausführungsform eines erfindungsgemäßen Systems ermöglicht eine Verlängerung oder Erweiterung einer solchen Vorrichtung oder Integration zusätzlicher, aktuierbarer Freiheitsgrade. Mit anderen Worten kann dadurch auch ein System aus einer erfindungsgemäßen Grundstruktur gebildet werden. Ein derart verlängertes bzw. erweitertes Instrument ermöglicht längere Wege (bspw. in einem Organ oder Rohrsystem). Zusätzliche aktuierbare Freiheitsgrade ermöglichen darüber hinaus bessere Manipulierbarkeit und höhere Flexibilität eines derart gebildeten Instruments.

Eine Ausführungsform eines Verfahrens zum Betreiben der Vorrichtung gemäß einer der vorhergehenden Ausführungsformen oder des Systems der zuvor genannten Ausführungsform umfasst: Bereitstellen der Vorrichtung gemäß einer der vorhergehenden Ausführungsformen oder des Systems gemäß der zuvor genannten Ausführungsform, und Ansteuern zumindest eines Teiles des Licht-aktuierbaren Materials mindestens eines Aktuators mittels Applizieren von Licht über den Lichtleiter und/oder die ansteuerbare Lichtquelle zum jeweiligen Licht-aktuierbaren Material des jeweiligen Aktuators mit einer zuvor wählbaren Intensität, Polarisation und/oder Wellenlänge oder einem Spektrum derart, dass damit die Wirkrichtung des jeweiligen Aktuators gesteuert wird.

### Definitionen und Spezifikationen

Hierin ist unter dem Begriff "Grundstruktur" eine Struktur umfassend mindestens zwei Elementen zu verstehen. Diese kann als Träger für die Integration weiterer Elemente dienen, um der Grundstruktur weitere Funktionen hinzuzufügen bzw. diese mittels der Grundstruktur einzusetzen (bspw. durch eine kontrollierbare Positionierung). Darüber hinaus erfüllt sie grundlegende Funktionen und ist wiederum für die Erweiterbarkeit zu einem System ausgelegt.

Hierin ist unter dem Begriff "Element" ein Bauteil zu verstehen, dass aus einem Material oder Komposit, insbesondere einem Licht-aktuierbarem Material oder einem zumindest teilweise aus einem solchen Material gebildetem Komposit, oder aus einem nicht-aktuierbaren Material gebildet ist und eine einfache Form bildet, welche in seiner Form nicht in weitere Struktureinheiten unterteilt ist.

Eine typische Länge eines Elementes liegt in einem Bereich von 1 mm bis zu 20 cm. Das Material für solche Elemente kann optional als Folie, Beschichtung oder Grundmaterial (engl.: "bulk-material") hergestellt werden. Folglich weisen solche Elemente eine Höhe von 1 µm bis 10 mm auf.

Nicht-aktuierbare Elemente sind zumindest teilweise aus einem Material aus der Gruppe von Materialien gebildet oder weisen zumindest eines davon zumindest teilweise auf, wobei diese Gruppe von Materialien umfasst: Elastomere und gummielastische Polymere (z.B. PDMS, Silikon). Ein solches Element kann entweder aufgrund des verwendeten Materials selbst flexibel (bzw. elastisch) oder starr sein oder bspw. durch lokale Materialschwächung oder Einschnitte flexibel gebildet sein.

Ein solches nicht-aktuierbares Element weist zumindest eines der Materialien aus der Gruppe von Materialien auf oder ist zumindest teilweise daraus gebildet, wobei diese Gruppen umfasst: Poly(organo)siloxane (Silikone), Gewebe bspw. aus Polypropylen, Kollagen-basierte Werkstoffe (insbesondere für den Einwegeinsatz in der Medizin), Elastomere, Butadien-Kautschuk, Butylkautschuk, Ethylenvinyl-acetat, Ethylen-Propylen-Dien-Kautschuk, Fluorkautschuk, Naturkautschuk, Polyurethan, Chloropren-Kautschuk, Acrylnitril/chloriertes Polyethylen/Styrol, Ethylen-Ethylacrylat-Copolymer, Ethylen-Propylen-Copolymer, Acrylnitril/Butadien/Acrylat, Acrylnitril/ Methylmethacrylat, Isopren-Kautschuk, Polyisobutylen, Polyvinylbutyral, Styrol-Butadien-Kautschuk, Vinylchlorid/Ethylen und/oder Vinylchlorid/Ethylen/Methacrylat.

Licht-aktuierbare Elemente sind zumindest teilweise aus einem Material (oder Komposit, welches mindestens ein solches Material enthält) aus der Gruppe von licht-aktuierbaren Materialien gebildet oder weisen zumindest eines davon zumindest teilweise auf, wobei diese Gruppe von Materialien umfasst: Flüssigkristall-Polymere und Hydrogele, wie bspw. Netzwerke des Monomers 4'-acryloyloxybutyl 2,5-(4'-butyloxybenzoyloxy) benzoate und/oder Hydrogele, wie bspw. poly (pentafluoro-phenyl acrylate), in Kombination mit einem oder mehreren photoaktiven Vernetzern, wie bspw.: 4,4"-Bis[6-(acryloyloxy)hexyloxy]azobenzene, Azotolene, 5-Azido-2-nitrobenzoic acid N-hydroxysuccinimide ester, 4-Benzoylbenzoic acid N-succinimidyl ester, LC-SDA (NHS-LC-Diazirine) (succinimidyl 6-(4,4'-azipentanamido)hexanoate), 4-(N-Maleimido)benzophenone, 4-(4-(Prop-2-yn-1-yloxy)benzoyl)benzoic acid, SDA (NHS-Diazirine) (succinimidyl 4,4'-azipentanoate), SPB (succinimidyl-[4-(psoralen-8-yloxy)]butyrate), Sulfo-LC-SDA (Sulfo-NHS-LC-Diazirine) (sulfosuccinimidyl 6-(4,4'-azipentanamido)hexanoate und/oder Sulfo-SANPAH (sulfosuccinimidyl 6-(4'azido-2'-nitrophenylamino)hexanoate) .

Hierin ist unter dem Begriff "Gelenkelement", ein Element mit zumindest der Eigenschaft eine möglichst verlustfreie Rotation mindestens zweier anderer Elemente zu ermöglichen. Ein solches Gelenkelement bildet dabei ein diskretes Rotationszentrum oder auch eine kontinuierliche Biegung (bspw. Torsion, Rotation) innerhalb des Gelenkelementes.

Dabei kann eine Rotation bspw. durch eine Steigerung der Elastizität des Gelenkmaterials oder mittels Aufbau aus zwei gegeneinander rotierbaren Teilen bzw. Bauteilen, wie bspw. ein Lager, gebildet oder bereitgestellt werden.

Das Gelenkelement liegt dabei typischerweise in der Mitte und innerhalb eines Elementes. Dessen Einbringen (wie bspw. gemäß der DIN-Norm: DIN 8593 "Definition von Fügeverfahren", welche hiermit mit in die Beschreibung aufgenommen gilt) erfolgt bspw. durch mindestens ein Verfahren aus der Gruppe von Fügeverfahren, wobei diese Gruppe umfasst: Urformen, Eingießen während der Herstellung, Formschluss, Umformung der Elemente, An- und Einpressen und/oder Kleben.

Hierin sind unter dem Begriff "Lichtleiter" transparente Bauteile wie Fasern, Röhren, Schläuche oder Stäbe zu verstehen, die Licht über kurze oder lange Strecken transportieren. Im Folgenden schließt die Verwendung des Begriffes Lichtleiter mit ein, dass in diesen an einem Ende Licht aus einer Lichtquelle eingekoppelt wird.

Solche Lichtleiter weisen zumindest ein Lichtleitermaterial aus der Gruppe von Lichtleitermaterialien auf oder sind zumindest teilweise daraus gebildet, wobei diese Gruppe umfasst: Polymethylmethacrylat (PMMA), Polycarbonat (PC), Quarzglas und/oder auch Flüssigkeiten in Schläuchen. Bei diesen Materialien ist auch optional eine Dotierung möglich, wie bspw. mit Germanium oder Phosphor. Die typische Größe verwendbarer Lichtleiter liegt im Bereich zwischen 10pm bis 1 mm.

Hierin ist unter dem Begriff "integrierter Lichtleiter" oder "integrierte, ansteuerbaren Lichtquelle" ein Lichtleiter oder eine Lichtquelle zu verstehen, die derart eingerichtet, dass sie entweder Licht zum Licht-aktuierbaren Material leitet (z.B. Glasfaser) oder in der Nähe des Licht-aktuierbaren Materials Licht erzeugt (z.B. LED).

Das Integrieren des Lichtleiters in die Elemente einer erfindungsgemäßen Vorrichtung kann bspw. mittels einer Passung, einer formschlüssigen Aussparung, einem Fixieren mit Hilfe von Klebstoffen, einem Eingießen (Integrieren) in Licht-aktuierbares Material bei der Herstellung einer solchen Vorrichtung, einem Eingießen (Integration) in ein Trägermaterial bei der Herstellung einer solchen Vorrichtung oder einem Einschreiben von Lichtleitfähigen Strukturen in das Trägermaterial oder das Licht-aktuierbaren Material erfolgen.

Hierin ist unter dem Begriff "Lichtquelle" eine Vorrichtung zum Erzeugen von Licht aus direkt zugeführter Energie (bspw. Strom) oder zuvor gespeicherter Energie (bspw. Phosphoreszenz) zu verstehen, die eine Abgabe von Licht ermöglicht (bspw. Strahlung UV, VIS, IR - typischer in einem Bereich zwischen 300 nm und 1000 nm).

Solche Lichtquellen weisen zumindest eine Lichtquelle aus der Gruppe von Lichtquellen auf oder sind zumindest teilweise daraus gebildet, wobei diese Gruppe umfasst: LEDs, OLED, Laser-, Superlumineszensdioden, Festkörper-, CO₂- oder Excimerlaser, Elektroluminszenzfolien und/oder Gasentladung- oder Glühlampen.

Mittels einer Schaltung kann die Leistung der Lichtquelle gesteuert werden. Eine solche Schaltung ist so eingerichtet, dass eine Lichtquelle durch gezieltes Variieren der Energieversorgung (z.B. Spannung) die Leistung der Lichtquelle kontrolliert oder/und die Eigenschaften des Lichts (z.B. Wellenlänge, Spektrum, Polarisation) reguliert. Eine solche Schaltung umfasst bspw.: eine Energieversorgung, wie bspw. eine Spannungsquelle, einem digitalen Eingang für ein Steuersignal, hierbei kann auf bestehende Standards zugriffen werden, wie u.a. bspw. CAN, I2C, SPI, USB, etc., sowie eine Logikschaltung welche den Verlauf der Leistung, Wellenlänge, Spektrum oder Polarisation der Lichtquelle zeitabhängig regulieren kann bspw. durch Pulsweitenmodulation.

Lichtquellen können dabei auch integrierte Lichtquellen sein, d.h. sie sind in das Element eingebettet bzw. integriert. Das Integrieren der Lichtquellen in die Elemente einer erfindungsgemäßen Vorrichtung kann bspw. mittels einer Passung, einer formschlüssigen Aussparung, einem Fixieren mit Hilfe von Klebstoffen, einem Eingießen (Integrieren) in Licht-aktuierbares Material bei der Herstellung einer solchen Vorrichtung, einem Eingießen (Integration) in ein Trägermaterial bei der Herstellung einer solchen Vorrichtung erfolgen. Hierfür geeignete kommerzielle Lichtquellen sind u.a. insbesondere: LEDs, OLEDs, Laser-, Superlumineszensdioden und Elektroluminszenzfolien, aufgrund ihrer kompakten Bauweise.

Hierin ist unter dem Begriff "Licht-aktuierbares Material" ein Material zu verstehen, bei dem als Reaktion auf ein oder mehrere Stimuli, insbesondere Licht, eine Wirkrichtung auf eine kontrollierbare und nutzbare Weise erzeugt wird. Bei Licht-aktuierbarem Material ist dieser Stimulus direkt Licht aus dem Ultraviolett-(UV), Sichtbaren- (VIS) oder Infrarot- (IR) Bereich (mit veränderbaren Wellenlängen, Intensität, oder Polarisation).

Ein solches Material besteht aus einem Basismaterial welches in der Lage ist, seine makromolekulare Struktur zu verändern. Diese Materialien sind bspw. Flüssigkristall-Polymere, wie bspw. Netzwerke des Monomers 4'-acryloyloxybutyl 2,5-(4'-butyloxybenzoyloxy) benzoate und/oder Hydrogele, wie bspw. poly (pentafluoro-phenyl acrylate).

Für die Aktuierung durch Licht müssen photoaktive Vernetzer mit eingebracht werden. Solche Vernetzer weisen zumindest ein Material aus der Gruppe von Vernetzermaterialien auf oder sind zumindest teilweise daraus gebildet, wobei diese Gruppe umfasst: Azofarbstoffe [24,25], wie bspw.: 4,4"-Bis[6-(acryloyloxy)hexyloxy]azobenzene, Azotolene, 5-Azido-2-nitrobenzoic acid N-hydroxysuccinimide ester, 4-Benzoylbenzoic acid N-succinimidyl ester, LC-SDA (NHS-LC-Diazirine) (succinimidyl 6-(4,4'-azipentanamido)hexanoate), 4-(N-Maleimido)benzophenone, 4-(4-(Prop-2-yn-1-yloxy)benzoyl)benzoic acid, SDA (NHS-Diazirine) (succinimidyl 4,4'-azipentanoate), SPB (succinimidyl-[4-(psoralen-8-yloxy)]butyrate), Sulfo-LC-SDA (Sulfo-NHS-LC-Diazirine) (sulfosuccinimidyl 6-(4,4'-azipentanamido)hexanoate und/oder Sulfo-SANPAH (sulfosuccinimidyl 6-(4'-azido-2'-nitrophenylamino)hexanoate) .

Demgegenüber ist hierin unter einem "Nicht Licht-aktuierbarem Material" ein Material zu verstehen, dass sich gegenüber einem Licht-Stimulus passiv verhält.

Für das Ausbilden einer Wirkrichtung ist eine Ausrichtung der Makromoleküle im jeweiligen Material von Nöten. Möglichkeiten zur Strukturierung hierfür sind bspw.: Magnetische Ausrichtung oder eine Beschichtung strukturierter Trägerflächen.

Beispiele für die Herstellung solcher Materialien finden sich bspw. in [26] und [27].

Ein Element oder Gelenkelement, das zumindest teilweise ein Licht-aktuierbares Material aufweist oder daraus zumindest teilweise gebildet ist, weist zumindest teilweise ein Material aus der Gruppe von Licht-aktuierbaren Materialien auf oder ist zumindest teilweise daraus gebildet, wobei diese Gruppe folgende Licht-aktuierbaren Materialien umfasst: Flüssigkristallpolymere (LCE), Hydrogele.

Hierin ist unter dem Begriff "Wirkrichtung" die Richtung zu verstehen, die aufgrund einer Licht-Stimulation von Licht-aktuierbarem Material eines Elements oder Gelenkelements hervorgerufen wird. Dies kann bspw. die Richtung einer der folgenden Wirkungen sein: Beschleunigung, Kraft, Bewegung, Dehnung, Expansion, Stauchung oder Positionsänderung.

Die Wirkrichtung kann in eine oder mehrere Dimensionen erfolgen: Torsion, Biegung, Translation.

Hierin ist unter dem Begriff "Komposit" ein Verbundwerkstoff oder Kompositwerkstoff (kurz Komposit, englisch: "composite" [material]) zu verstehen. Komposit ist ein Werkstoff aus zwei oder mehr verbundenen Materialien, der andere Werkstoffeigenschaften besitzt als seine einzelnen Komponenten. Für die Eigenschaften der Verbundwerkstoffe sind stoffliche Eigenschaften und Geometrie der Komponenten von Bedeutung. Insbesondere spielen oft Größeneffekte eine Rolle. Die Verbindung erfolgt durch Stoff- oder Formschluss oder eine Kombination von beidem. Verbundwerkstoffe sind Gemische aus sortenreinen Grundstoffen. Eine Lösung der einzelnen Grundstoffe untereinander findet dabei nicht oder nur oberflächlich statt. Durch die Compoundierung werden somit mindestens zwei Stoffe miteinander verbunden. Ziel der Compoundierung ist es, einen Werkstoff zu erhalten, der besonders günstige Eigenschaften der Bestandteile kombiniert. Meist ist es von Bedeutung, eine innige Verbindung der Phasen auch langfristig und unter Belastung sicherstellen zu können. Die Ziele der Compoundierung sind vielfältig und richten sich nach den gewünschten Eigenschaften des späteren Bauteils. Typische Ziele der Compoundierung sind: Veränderung der mechanischen Eigenschaften eines Bindemittels (Grundpolymer).

Ein Komposit kann hier eine Zusammensetzung aus mindestens zwei Materialien sein. Diese Materialien umfassen mindestens ein Licht-aktuierbares Material und optional nicht-aktuierbare Materialien oder Materialien, welche durch einen anderen Stimulus (bspw. Wärme, pH-Wert, Spannung, ...) aktuierbar sind.

Unter dem Begriff "Elastizität" ist hierin die Eigenschaft eines Elementes, Körpers oder Werkstoffes, unter Krafteinwirkung seine Form zu verändern und bei Wegfall der einwirkenden Kraft in die Ursprungsform zurückzukehren, zu verstehen.

Unter dem Begriff "Materialschwächung" ist hierin eine Strukturveränderung zur Erzeugung niedrigerer Elastizität eines Elementes zu verstehen. Solch eine Schwächung des Materials kann bspw. mittels Einschnitt oder Einschnitten, abschnittsweise dünnere Materiallagen, Netzartige Strukturierung oder Porositäten des Materials und/oder Aussparungen gebildet sein.

Als "Aktuator", auch "Aktor", wird eine antriebstechnische Baueinheit bezeichnet und ist hierin auch darunter zu verstehen, die ein Signal in mechanische Bewegungen umsetzen und damit aktiv in den gesteuerten Prozess eingreift. Ihre Aufgabe ist die kontrollierte Verursachung einer Wirkrichtung.

Als System wird im Allgemeinen ein abgrenzbares, natürliches oder künstliches "Gebilde" bezeichnet und ist auch hierin darunter zu verstehen, das aus verschiedenen Komponenten besteht, die aufgrund bestimmter geordneter Beziehungen untereinander als gemeinsames Ganzes betrachtet werden können. Mit anderen Worten, umfasst hierin ein System zumindest eine Anordnung gebildet aus mindestens zwei erfindungsgemäßen Vorrichtungen die wiederum zumindest jeweils eine erfindungsgemäße Grundstruktur aufweisen, wobei jedoch optional ein erfindungsgemäßes System auch noch zusätzliche Elemente, Komponenten oder Bauteile aufweisen kann. Solche zusätzlichen Elemente, Komponenten oder Bauteile können bspw. Grundstrukturen ohne Licht-aktuierbares Element oder mit einem über andere Stimuli (bspw. Wärme, pH-Wert, Spannung, ...) aktuierbare Elemente sein. Einzelne Grundstruktur-Elemente können durch Kleben oder durch verschiedene weitere Fügetechniken (wie bspw. gemäß der DIN-Norm: DIN 8593 "Definition von Fügeverfahren", welche hiermit mit in die Beschreibung aufgenommen gilt) verbunden werden bspw. durch mindestens ein Verfahren aus der Gruppe von Fügeverfahren, wobei diese Gruppe umfasst: Urformen, Eingießen während der Herstellung, Formschluss, Umformung der Elemente, An- und Einpressen und/oder Kleben.

Ein medizinischer Kontinuumsroboter bezeichnet eine ansteuerbare Struktur, optional mit integriertem Werkzeug und/oder Sensorik. Ihr flexibler Körper kann entlang kontinuierlicher Kurven bewegt werden. Aufgrund der Vielzahl von Freiheitsgraden und der Möglichkeit zur Miniaturisierung, bieten Kontinuumsroboter die Möglichkeit zu chirurgischen minimal-invasiven Anwendungen mit erhöhter Sicherheit und bessere Erreichbarkeit tiefliegender Pathologien durch das Umgehen von Hindernissen.

### Kurzbeschreibung der Figuren

Weitere Einzelheiten und Merkmale der vorliegenden Erfindung ergeben sich aus der nachfolgenden Beschreibung eines bevorzugten Ausführungsbeispiels, insbesondere in Verbindung mit den abhängigen Ansprüchen. Hierbei können die jeweiligen Merkmale für sich alleine oder zu mehreren in Kombination miteinander verwirklicht sein. Die Erfindung ist nicht auf die Ausführungsbeispiele bzw. -formen beschränkt.

Die Ausführungsbeispiele bzw. -formen sind schematisch in den nachfolgenden Figuren dargestellt. Hierbei bezeichnen gleiche Bezugsziffern in den Figuren gleiche oder funktionsgleiche Elemente bzw. hinsichtlich ihrer Funktionen einander entsprechende Elemente.

Zur Veranschaulichung und ohne einschränkende Wirkung ergeben sich weitere Merkmale und Vorteile der Erfindung aus der Beschreibung der beigefügten Zeichnungen. Darin zeigt:
- Fig. 1: schematische Querschnittsdarstellungen dreier Ausführungsformen erfindungsgemäßer Vorrichtungen der Grundstrukturen A, B und C;
- Fig. 2: schematische Querschnittsdarstellungen dreier Ausführungsformen erfindungsgemäßer Vorrichtungen der Grundstrukturen D, E und F;
- Fig. 3: in drei Subfiguren, 3A, 3B und 3C, schematische Querschnittsdarstellungen dreier weiterer Ausführungsformen erfindungsgemäßer Vorrichtungen;
- Fig. 4: in drei Subfiguren, 4A, 4B und 4C, schematische Querschnittsdarstellungen dreier weiterer Ausführungsformen erfindungsgemäßer Vorrichtungen;
- Fig. 5: in den drei Subfiguren, 5A, 5B und 5C, schematische Querschnittsdarstellungen dreier weiterer Ausführungsformen erfindungsgemäßer Vorrichtungen;
- Fig. 6: schematische Querschnittsdarstellungen von vier weiteren Ausführungsformen erfindungsgemäßer Vorrichtungen;
- Fig. 7: schematische Querschnittsdarstellungen von zwei weiteren Ausführungsformen, dargestellt in den beiden Subfiguren 7A und 7B, erfindungsgemäßer Vorrichtungen;
- Fig. 8: eine schematische Querschnittsansicht einer Ausführungsform eines erfindungsgemäßen Systems im Ruhezustand A und im aktuierten Zustand B;
- Fig. 9: eine schematische Querschnittsansicht einer weiteren Ausführungsform eines erfindungsgemäßen Systems im Ruhezustand A und im aktuierten Zustand B; sowie
- Fig. 10: eine schematische Darstellung einer Ausführungsform eines erfindungsgemäßen Verfahrens.

### Detaillierte Beschreibung

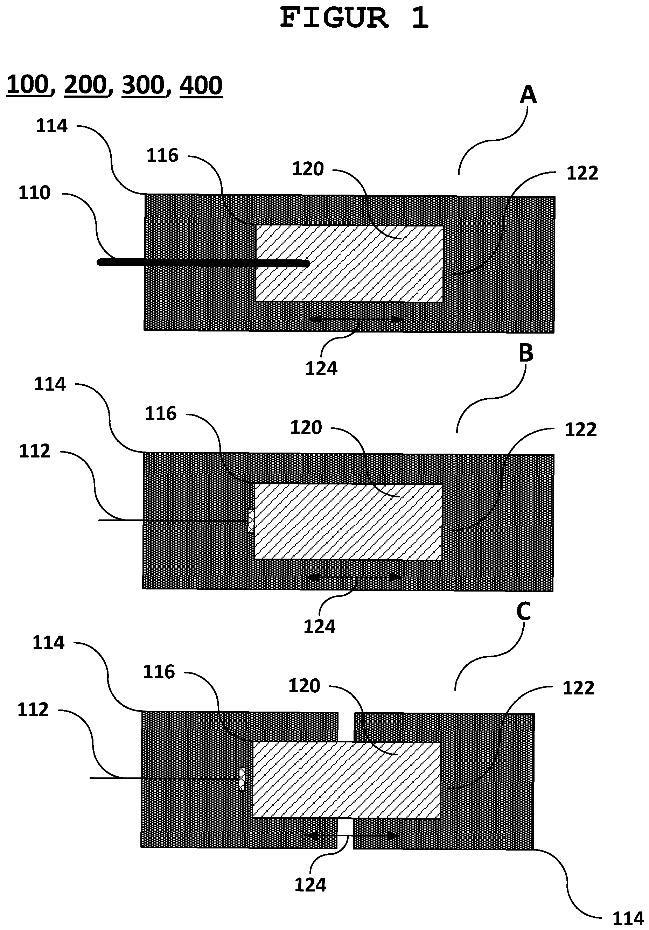

Fig. 1 zeigt drei schematische Querschnittsdarstellungen dreier Ausführungsformen erfindungsgemäßer Vorrichtungen der Grundstrukturen A, B und C.

Die Grundstruktur A integriert den Lichtleiter 110 in das Licht-aktuierbare Element 116 aus Licht-aktuierbarem Material 120. In dieser Ausführungsform umschließt das nicht-aktuierbare Element 114 das Licht-aktuierbare Element 116 vollständig. Die Wirkrichtung 124 ist eine Dehnung oder Biegung des Licht-aktuierbaren Materials auf einer Seite der Grundstruktur. Der auf diese Weise gebildete Aktuator 122 erzeugt somit eine einseitige Dehnung.

Die Grundstruktur B integriert die ansteuerbare Lichtquelle 112 direkt an das Licht-aktuierbare Element 116 aus Licht-aktuierbarem Material 120. In dieser Ausführungsform umschließt das nicht-aktuierbare Element 114 das Licht-aktuierbare Element 116 vollständig. Die Wirkrichtung 124 ist eine Dehnung des Licht-aktuierbaren Materials auf einer Seite der Grundstruktur. Der auf diese Weise gebildete Aktuator 122 erzeugt somit eine einseitige Dehnung oder Biegung.

Die Grundstruktur C integriert die ansteuerbare Lichtquelle 112 oder einen Lichtleiter 110 an das Licht-aktuierbare Element 116 aus Licht-aktuierbarem Material 120. Das Licht aus der ansteuerbaren Lichtquelle 112 (und/oder alternativ oder zusätzlich zumindest ein Lichtleiter 110) verläuft abschnittsweise durch das nicht-aktuierbare Material. In dieser Ausführungsform umschließt das nicht-aktuierbare Element 114 das Licht-aktuierbare Element 116 nur teilweise. Es liegt seitlich oder in der Mitte des Licht-aktuierbaren Elements 116 ein freiliegender Abschnitt vor, d.h. ein Bereich der frei von Licht-aktuierbarem Material ist. Die Wirkrichtung 124 ist eine Dehnung des Licht-aktuierbaren Materials auf einer Seite der Grundstruktur. Der auf diese Weise gebildete Aktuator 122 erzeugt somit eine einseitige Dehnung oder Biegung.

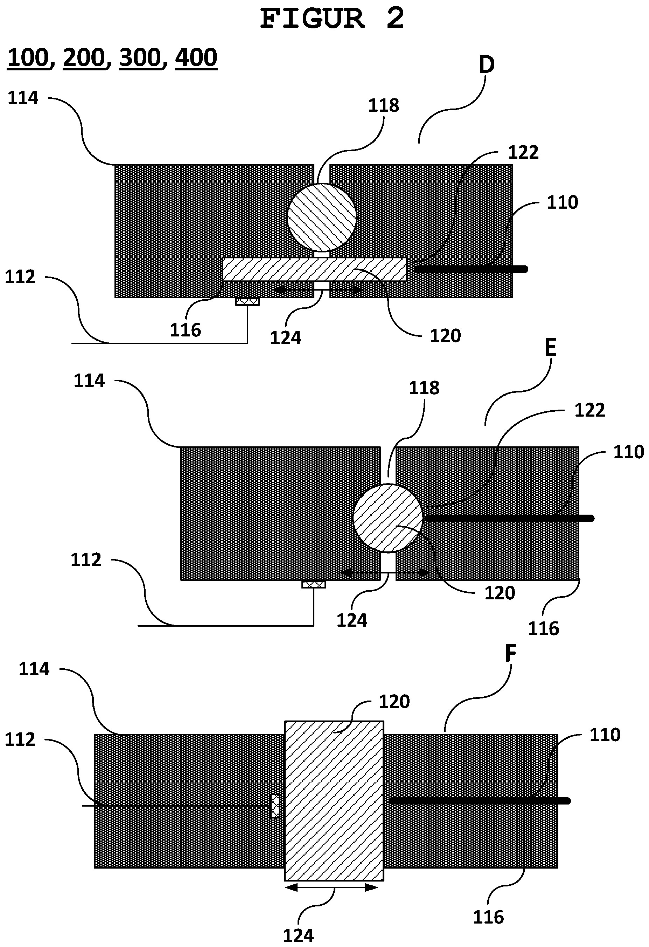

Fig. 2 zeigt drei schematische Querschnittsdarstellungen dreier Ausführungsformen erfindungsgemäßer Vorrichtungen der Grundstrukturen D, E und F.

Die Grundstruktur D integriert die ansteuerbare Lichtquelle 112 am Rand des nicht-aktuierbaren Elements 114 und/oder einen Lichtleiter 110. Diese ermöglichen die Steuerung des Licht-aktuierbaren Materials 120 im Element 116. Das nicht-aktuierbare Element 114 ist in der Mitte durch ein Gelenkelement 118 beweglich gestaltet, so dass die Wirkrichtung 124 des Aktuators 122 eine Rotation um dieses Gelenkelement 118 bewirkt.

Die Grundstruktur E integriert die ansteuerbare Lichtquelle 112 am Rand des nicht-aktuierbaren Elements 114 und/oder einen Lichtleiter 110. Diese ermöglichen die Steuerung des Licht-aktuierbaren Materials 120 im Gelenkelement 118. Das Gelenkelement ist aus Licht-aktuierbarem Material geformt, so dass die Wirkrichtung 124 des Aktuators 122 eine Rotation von Element 114 gegen Element 116 um dieses Gelenkelement 118 bewirkt.

Die Grundstruktur F integriert die ansteuerbare Lichtquelle 112 durch das eine Element 114 hindurch bis an das Licht-aktuierbare Material 120. Alternativ oder zusätzlich ist durch das Element 116 ein Lichtleiter 110 bis an das Licht-aktuierbare Material 120 integriert. Das Licht-aktuierbare Material 120 kann sich ausdehnen und erzeugt bspw. bei homogener Ausdehnung eine translatorische Bewegung der beiden Elemente 114 und 116 zueinander.

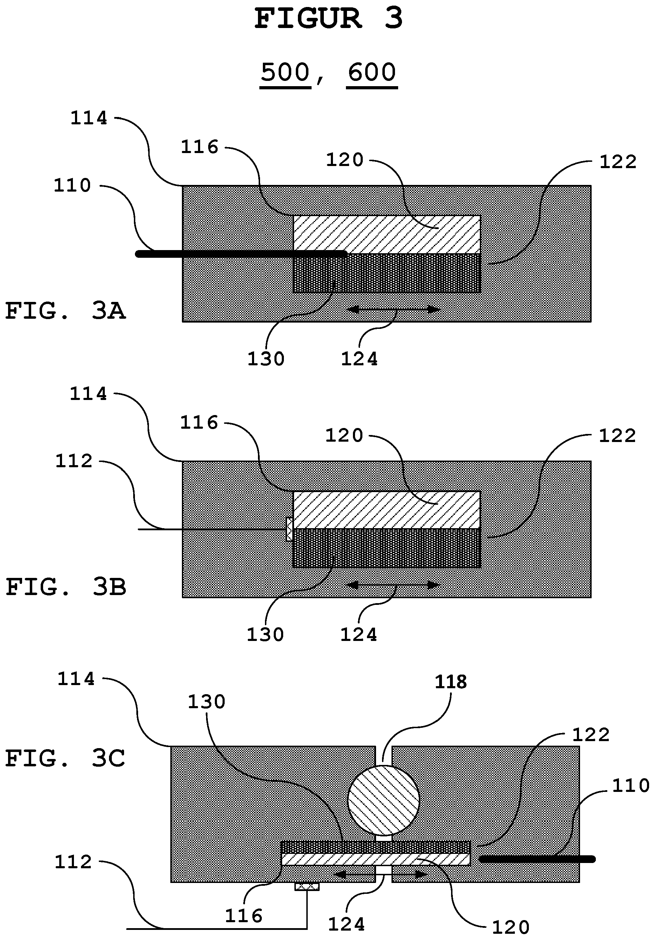

Fig. 3 zeigt in drei Subfiguren, d.h. hier Subfigur 3A, Subfigur 3B und Subfigur 3C, drei schematische Querschnittsdarstellungen dreier weiterer Ausführungsformen erfindungsgemäßer Vorrichtungen.

Die Grundstruktur A integriert den Lichtleiter 110 in das Licht-aktuierbare Element 116 aus einem Komposit, welches aus zwei verschieden aktuierbaren Materialien 120 oder aus einem Licht-aktuierbaren Material 120 und einem weiteren Material 130 mit anpassbarer Steifigkeit besteht. In dieser Ausführungsform umschließt das nicht-aktuierbare Element 114 das Licht-aktuierbare Element 116 vollständig. Die Wirkrichtung 124 ist eine Dehnung des Licht-aktuierbare Materials an der oberen Seite während das zweite Kompositmaterial die Dehnung nicht oder nicht vollständig mitvollzieht. Der auf diese Weise gebildete Aktuator 122 kann somit eine Biegung in eine Richtung erzeugen.

Die Grundstruktur B integriert die ansteuerbare Lichtquelle 112 oder einen Lichtleiter direkt an das Licht-aktuierbare Element 116 aus einem Komposit, welches aus zwei verschieden aktuierbaren Materialien 120 oder aus einem Licht-aktuierbarem Material 120 und einem weiteren Material 130 mit anpassbarer Steifigkeit besteht. In dieser Ausführungsform umschließt das nicht-aktuierbare Element 114 das Licht-aktuierbare Element 116 vollständig. Die Wirkrichtung 124 ist eine Dehnung des Licht-aktuierbare Materials an der oberen Seite während das zweite Kompositmaterial die Dehnung nicht oder nicht vollständig mitvollzieht. Der auf diese Weise gebildete Aktuator 122 kann somit eine Biegung nach oben erzeugen.

Die Grundstruktur C integriert die ansteuerbare Lichtquelle 112 am äußeren Rand des nicht-aktuierbaren Elements 114 sowie von der anderen Seite einen Lichtleiter 110. Diese ermöglichen die Steuerung des Elements 116 bestehend aus einem Komposit, welches aus zwei verschieden Licht-aktuierbaren Materialien 120 oder aus einem Licht-aktuierbaren Material 120 und einem weiteren Material 130 mit anpassbarer Steifigkeit besteht. Das nicht-aktuierbare Element 114 ist in der Mitte durch ein Gelenkelement 118 beweglich gestaltet, so dass die Wirkrichtung 124 des Aktuators 122 eine Rotation um dieses Gelenkelement 118 bewirkt.

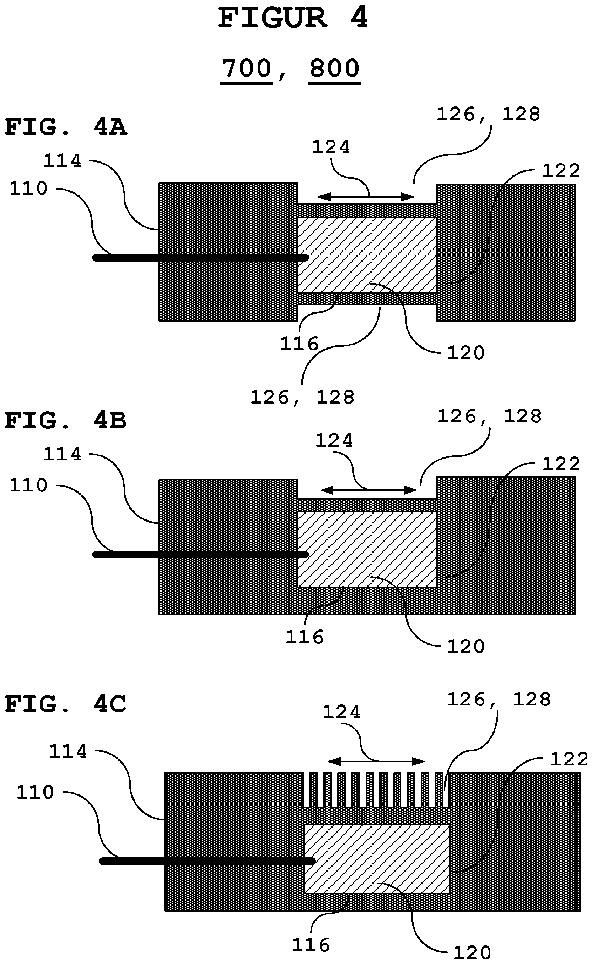

Fig. 4 zeigt in drei Subfiguren, d.h. hier Subfigur 4A, Subfigur 4B und Subfigur 4C, drei schematische Querschnittsdarstellungen dreier weiterer Ausführungsformen erfindungsgemäßer Vorrichtungen.

Die Grundstruktur A integriert den Lichtleiter 110 oder eine ansteuerbare Lichtquelle in das Licht-aktuierbare Element 116 aus Licht-aktuierbarem Material 120. In dieser Ausführungsform umschließt das nicht-aktuierbare Element 114 das Licht-aktuierbare Element 116 vollständig. Ein oder mehrere Einschnitte 126 im Element 114 erzeugen eine lokale Materialschwächung 128 auf beiden Seiten. Auf diese Weise erzeugt die Wirkrichtung (bspw. eine Dehnung oder Biegung) des Aktuators 122 eine stärkere Bewegung des Elements 114.

Die Grundstruktur B integriert den Lichtleiter 110 oder eine ansteuerbare Lichtquelle in das Licht-aktuierbare Element 116 aus Licht-aktuierbarem Material 120. In dieser Ausführungsform umschließt das nicht-aktuierbare Element 114 das Licht-aktuierbare Element 116 vollständig. Ein oder mehrere einseitige Einschnitte 126 im Element 114 erzeugen eine lokale Materialschwächung 128 auf nur einer Seite. Auf diese Weise erzeugt die Wirkrichtung (bspw. eine Dehnung oder Biegung) des Aktuators 122 eine stärkere Bewegung des Elements 114 an der Seite der Materialschwächung.

Die Grundstruktur C integriert den Lichtleiter 110 oder eine ansteuerbare Lichtquelle in das Licht-aktuierbare Element 116 aus Licht-aktuierbarem Material 120. In dieser Ausführungsform umschließt das nicht-aktuierbare Element 114 das Licht-aktuierbare Element 116 vollständig. Viele kleine Einschnitte 126 im Element 114 erzeugen eine lokale Materialschwächung 128 auf nur einer Seite. Auf diese Weise erzeugt die Wirkrichtung (bspw. eine Dehnung oder Biegung) des Aktuators 122 eine stärkere Bewegung des Elements 114 an der oberen als an Seite der Materialschwächung.

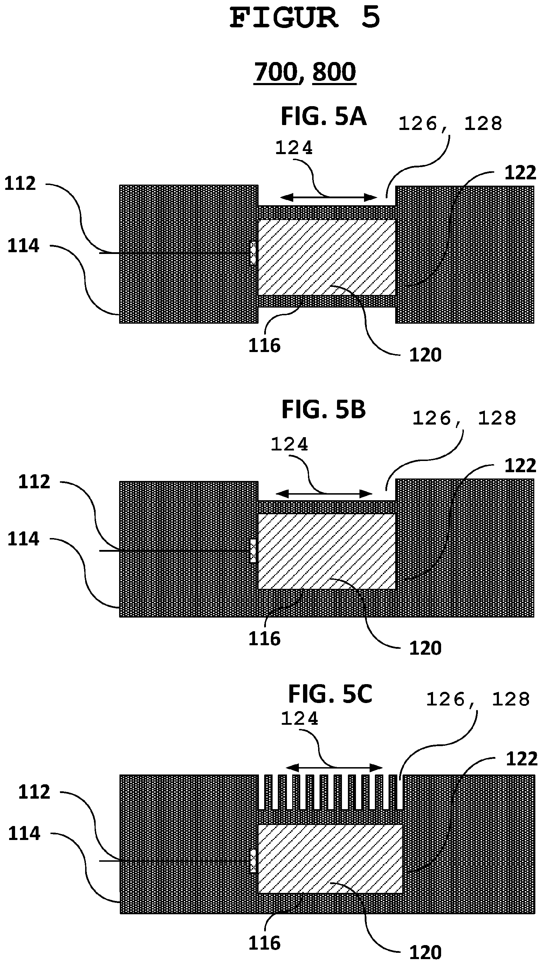

Fig. 5 zeigt in den drei Subfiguren, d.h. hier Subfigur 5A, Subfigur 5B und Subfigur 5C, drei schematische Querschnittsdarstellungen dreier weiterer Ausführungsformen erfindungsgemäßer Vorrichtungen.

Die Grundstruktur A integriert die ansteuerbare Lichtquelle 112 oder einen Lichtleiter direkt an das Licht-aktuierbare Element 116 aus Licht-aktuierbare Material 120. In dieser Ausführungsform umschließt das nicht-aktuierbare Element 114 das Licht-aktuierbare Element 116 vollständig. Ein Einschnitt 126 im Element 114 erzeugen eine lokale Materialschwächung 128 auf beiden Seiten. Auf diese Weise erzeugt die Wirkrichtung (bspw. eine Dehnung oder Biegung) des Aktuators 122 eine stärkere Bewegung des Elements 114.

Die Grundstruktur B integriert die ansteuerbare Lichtquelle 112 oder einen Lichtleiter direkt an das Licht-aktuierbare Element 116 aus Licht-aktuierbarem Material 120. In dieser Ausführungsform umschließt das nicht-aktuierbare Element 114 das Licht-aktuierbare Element 116 vollständig. Ein einseitiger Einschnitt 126 im Element 114 erzeugen eine lokale Materialschwächung 128 auf nur einer Seite. Auf diese Weise erzeugt die Wirkrichtung (bspw. eine Dehnung oder Biegung) des Aktuators 122 eine stärkere Bewegung des Elements 114 an der oberen als an der unteren Seite.

Die Grundstruktur C integriert die ansteuerbare Lichtquelle 112 oder einen Lichtleiter direkt an das Licht-aktuierbare Element 116 aus Licht-aktuierbarem Material 120. In dieser Ausführungsform umschließt das nicht-aktuierbare Element 114 das Licht-aktuierbare Element 116 vollständig. Viele kleine Einschnitte 126 im Element 114 erzeugen eine lokale Materialschwächung 128 auf nur einer Seite. Auf diese Weise erzeugt die Wirkrichtung (bspw. eine Dehnung oder Biegung) des Aktuators 122 eine stärkere Bewegung des Elements 114 an Seite der Materialschwächung.

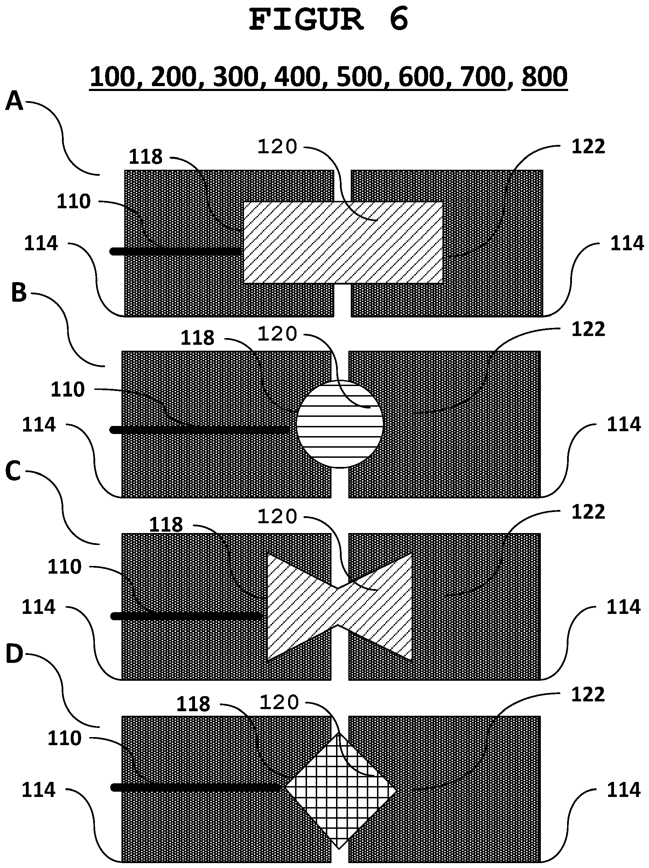

Fig. 6 zeigt vier schematische Querschnittsdarstellungen von vier weiteren Ausführungsformen erfindungsgemäßer Vorrichtungen. Es sind verschiedene Formausführungen des Aktuators 122 dargestellt.

In Grundstruktur A umschließt das nicht-aktuierbare Element 114 das Licht-aktuierbare Element 116 aus Licht-aktuierbarem Material 120 nur teilweise. Es liegt in der Mitte des Licht-aktuierbaren Elements 116 ein freiliegender Abschnitt vor. Zwischen beiden Elementen ist ein Gelenkelement 118 mit rechteckigem Querschnitt. Das Gelenkelement 118 bildet den Aktuator 120. Ein Lichtleiter 110 oder eine ansteuerbare Lichtquelle ist an das Licht-aktuierbare Element 118 integriert.

In Grundstruktur B umschließt das nicht-aktuierbare Element 114 das Licht-aktuierbare Element 116 aus Licht-aktuierbarem Material 120 nur teilweise. Es liegt in der Mitte des Licht-aktuierbaren Elements 116 ein freiliegender Abschnitt vor. Zwischen beiden Elementen ist ein Gelenkelement 118 mit kreisförmigem Querschnitt. Das Gelenkelement 118 bildet den Aktuator 120. Ein Lichtleiter 110 oder eine ansteuerbare Lichtquelle ist an das Licht-aktuierbare Element 118 integriert.

In Grundstruktur C umschließt das nicht-aktuierbare Element 114 das Licht-aktuierbare Element 116 aus Licht-aktuierbarem Material 120 nur teilweise. Es liegt in der Mitte des Licht-aktuierbaren Elements 116 ein freiliegender Abschnitt vor. Zwischen beiden Elementen ist ein Gelenkelement 118 mit einem Querschnitt welcher einen Formschluss erlaubt. Das Gelenkelement 118 bildet den Aktuator 120. Ein Lichtleiter 110 oder eine ansteuerbare Lichtquelle ist an das Licht-aktuierbare Element 118 integriert.

In Grundstruktur D umschließt das nicht-aktuierbare Element 114 das Licht-aktuierbare Element 116 aus Licht-aktuierbarem Material 120 nur teilweise. Es liegt in der Mitte des Licht-aktuierbaren Elements 116 ein freiliegender Abschnitt vor. Zwischen beiden Elementen ist ein Gelenkelement 118 mit quadratischem oder rautenförmigem Querschnitt. Das Gelenkelement 118 bildet den Aktuator 120. Ein Lichtleiter 110 oder eine ansteuerbare Lichtquelle ist an das Licht-aktuierbare Element 118 integriert.

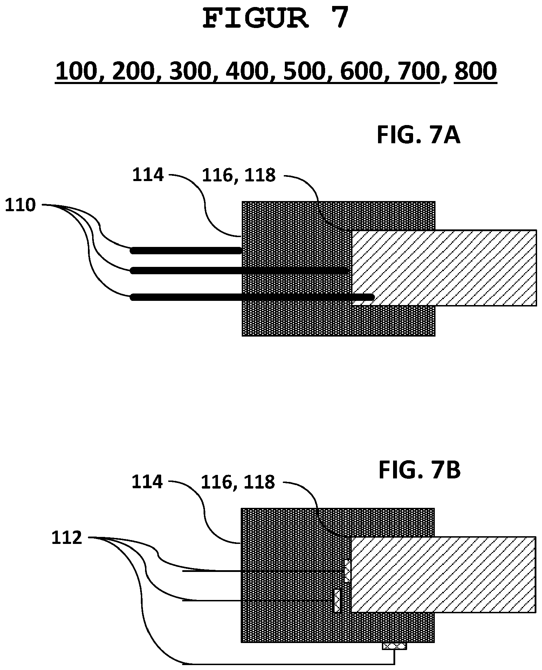

Fig. 7 zeigt zwei schematische Querschnittsdarstellungen von zwei weiteren Ausführungsformen von teilweisen, erfindungsgemäßen Vorrichtungen. D.h. die Subfigur 7A stellt Ausführungsbeispiele von Lichtleiteranordnungen dar und die Subfigur 7B stellt Ausführungsbeispiele von Lichtquellenanordnungen dar. Es sind jeweils verschiedene Möglichkeiten gezeigt, Licht in das Licht-aktuierbare oder zum Licht-aktuierbaren Material zu bringen bzw. zu leiten.

In der Grundstruktur, die schematisch in Subfigur 7A dargestellt ist, umschließt das nicht-aktuierbare Element 114 das Licht-aktuierbare Element 116 aus Licht-aktuierbarem Material 120 welches auch Gelenkelement 118 sein kann nur teilweise. Drei Lichtleiter 110 zeigen mögliche Ausführungsformen: 1) Ein Lichtleiter wird nur bis an den äußeren Rand des Elementes 116 geführt und das Licht wird durch dieses Element 116 bis in das Licht-aktuierbaren Element 116 geleitet. 2) Ein Lichtleiter wird durch das nicht-aktuierbare Element 114 nur bis an den äußeren Rand des Licht-aktuierbaren Elementes 116 geführt und das Licht wird durch dieses Element 116 bis in das Licht-aktuierbare Element 116 geleitet. 3) Ein Lichtleiter wird durch das nicht-aktuierbare Element 114 bis in das Licht-aktuierbare Element 116 hineingeführt und das Licht breitet sich direkt in diesem Element 116 aus.

In der Grundstruktur, die schematisch in Subfigur 7B dargestellt ist, umschließt das nicht-aktuierbare Element 114 das Licht-aktuierbare Element 116 aus Licht-aktuierbarem Material 120 welches auch Gelenkelement 118 sein kann nur teilweise. Drei ansteuerbare Lichtquellen 110 zeigen mögliche Ausführungsformen: 1) Eine ansteuerbare Lichtquelle wird nur bis an den äußeren Rand des Elementes 116 geführt und das Licht wird durch dieses Element 116 bis in das Licht-aktuierbaren Element 116 geleitet. 2) Eine ansteuerbare Lichtquelle wird durch das nicht-aktuierbare Element 114 nur bis vor das Licht-aktuierbare Element 116 geführt und das Licht wird durch dieses Element 116 bis in das Licht-aktuierbare Element 116 geleitet. 3) Eine ansteuerbare Lichtquelle wird durch das nicht-aktuierbare Element 114 bis direkt an den Rand das Licht-aktuierbaren Elementes 116 geführt und das Licht breitet sich direkt in diesem Element 116 aus.

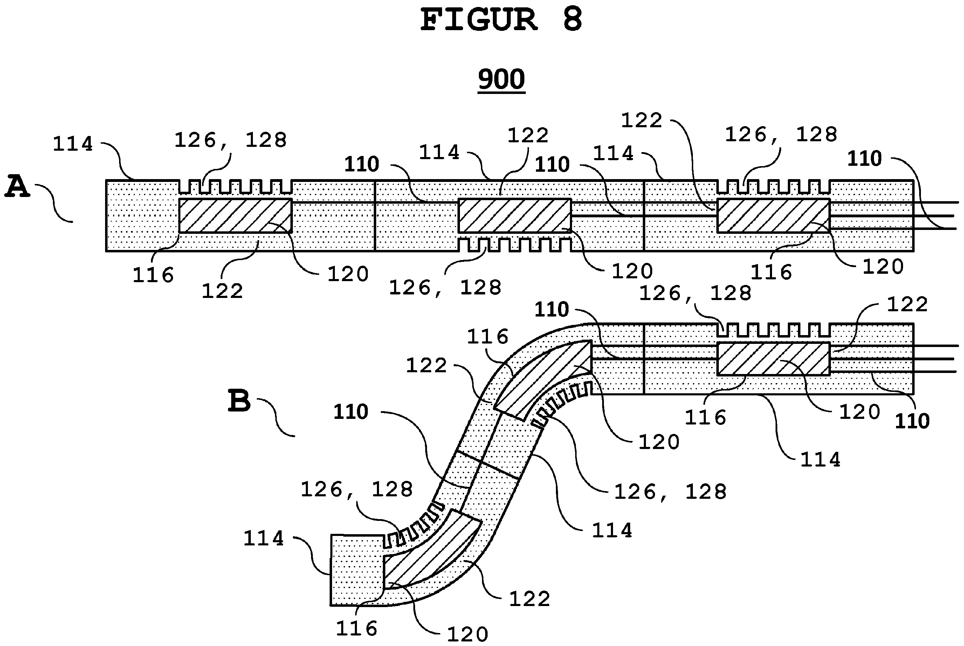

Fig. 8 zeigt eine schematische Querschnittsansicht einer Ausführungsform eines erfindungsgemäßen Systems im Ruhezustand A und im aktuierten Zustand B. Das System ist aus 3 gleichen Grundstrukturen zusammengesetzt (bspw. mittels Verkleben, wie bspw. mit Silikon/PMMA oder auf andere Weise (vgl. oben Definition des Systems) gefügt). Diese bestehen jeweils aus einem nicht-aktuierbaren Element 114 mit lokalen Materialschwächungen 128 durch Einschnitte 126 und einem Aktuator 122 aus einem Element 116 aus Licht-aktuierbarem Material 120. Jeder Aktuator 120 ist mit mindestens einem Lichtleiter 110 oder mindestens einer ansteuerbaren Lichtquelle verbunden. Die Lichtleiter 110 werden in der gezeigten Ausführungsform von einer Seite her in das System integriert. Diese Seite bildet somit die Basis des Roboters, nach mindestens einer aktuierbaren Grundstruktur (hier bspw. drei Grundstrukturen) wird die Spitze des Systems gebildet. Auch die Integration von mehreren und verschiedenen Seiten her ist möglich.

Im Ruhzustand A sind alle Aktuatoren 120 in geradliniger Haltung. Im aktuierten Zustand B sind die beiden Grundstrukturen an der Spitze aktuiert und erzeugen eine Biegung in die Richtung der lokalen Materialschwächung. Auf diese Weise kann die hier dargestellt "S"-Form erreicht werden, durch andere Anordnungen sind auch weitere Ausformungen wie "C"- oder mehrfach gekrümmte Formen erreichbar.

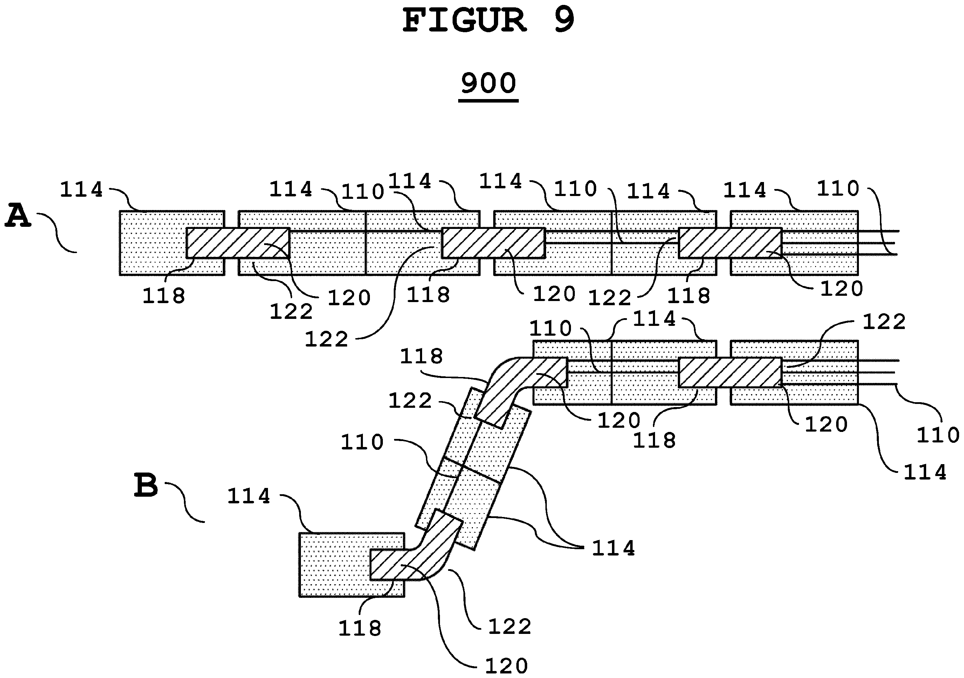

Fig. 9 zeigt eine schematische Querschnittsansicht einer Ausführungsform eines erfindungsgemäßen Systems im Ruhezustand A und im aktuierten Zustand B. Das System ist aus 3 gleichen Grundstrukturen zusammengesetzt. Diese bestehen jeweils aus zwei nicht-aktuierbaren Elementen 114 welche durch ein Gelenkelement 118 verbunden sind. Das Gelenkelement 118 ist aus Licht-aktuierbarem Material 120 und bildet den Aktuator 122. Jeder Aktuator 120 ist mit mindestens einem Lichtleiter 110 oder mindestens einer ansteuerbaren Lichtquelle verbunden. Die Lichtleiter 110 werden in der gezeigten Ausführungsform von einer Seite her in das System integriert. Diese Seite bildet somit die Basis des Roboters, nach mindestens einer aktuierbaren Grundstruktur (hier 3) wird die Spitze des Systems gebildet. Auch die Integration von mehreren und verschiedenen Seiten her ist möglich.

Im Ruhzustand A sind alle Aktuatoren 120 in geradliniger Haltung. Im aktuierten Zustand B sind die beiden Grundstrukturen an der Spitze aktuiert und erzeugen eine Biegung. Die Biegerichtung der Aktuatoren wird durch die Strukturierung des Licht-aktuierbaren Materials und die Ausrichtung des Elementes bestimmt.

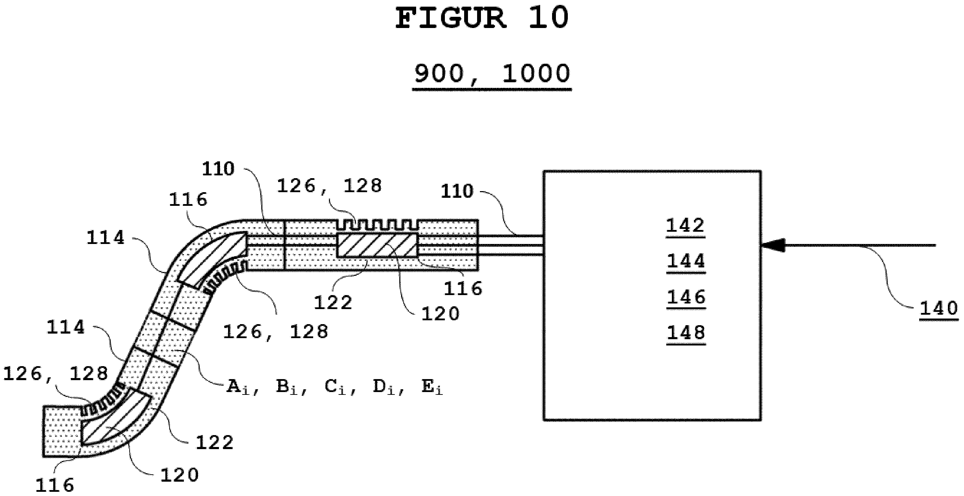

Fig. 10 zeigt eine schematische Darstellung einer Ausführungsform eines erfindungsgemäßen Verfahrens. Die Lichtansteuerung 140 steuert die Lichtintensität 142, Lichtpolarisation 144, Lichtwellenlänge 146 und Lichtspektrum 148 welche/welches in die Lichtleiter eingespeist wird um das System 900 zu aktuieren. Das System ist in aktuiertem Zustand dargestellt. Das System ist hier bspw. aus 3 gleichen Grundstrukturen zusammengesetzt. Diese bestehen jeweils aus einem nicht-aktuierbaren Element 114 mit lokalen Materialschwächungen 128 durch Einschnitte 126 und einem Aktuator 122 aus einem Element 116 aus Licht-aktuierbarem Material 120. Die Lichtleiter 110 oder ansteuerbaren Lichtquellen werden in der gezeigten Ausführungsform von einer Seite her in das System integriert. Diese Seite bildet somit die Basis des Roboters, nach mindestens einer aktuierbaren Grundstruktur (hier 3) wird die Spitze des Systems gebildet. Je nach Verwendungszweck können nicht-aktuierte Grundstrukturen Aᵢ, Bᵢ, Cᵢ, Dᵢ, Eᵢ und/oder Fᵢ ..., wobei der Index i = 1, 2, 3, ... eine beliebige natürliche Zahl repräsentiert, zwischen den aktuierbaren Grundstrukturen platziert werden. Auch die Integration der Lichtleiter von mehreren und verschiedenen Seiten her ist möglich. Im aktuierten Zustand sind die beiden Grundstrukturen an der Spitze aktuiert und erzeugen eine Biegung in die Richtung der lokalen Materialschwächung.

### Literaturliste

[1] ......... Vitiello, V., Kwok, K. W. & Yang, G. Z. Introduction to robot-assisted minimally invasive surgery (MIS). Med. Robot. Minim. Invasive Surg. 1-40 (2012). doi:10.1016/B978-0-85709-130-7.50001-6
[2] ......... Burgner-Kahrs, J., Rucker, D. C. & Choset, H. Continuum Robots for Medical Applications: A Survey. IEEE Trans. Robot. 31, 1261-1280 (2015)
[3] ......... Kato, T., Okumura, I., Kose, H., Takagi, K. & Hata, N. Extended kinematic mapping of tendon-driven continuum robot for neuroendoscopy BT - 2014 IEEE/RSJ International Conference on Intelligent Robots and Systems, IROS 2014, September 14, 2014 - September 18, 2014. 1997-2002 (2014). doi:10.1109/IROS.2014.6942828
[4] ......... Kutzer, M. D. M. et al. Design of a new cable-driven manipulator with a large open lumen: Preliminary applications in the minimally-invasive removal of osteolysis. Proc. - IEEE Int. Conf. Robot. Autom. 2913-2920 (2011). doi:10.1109/ICRA.2011.5980285
[5] ......... Ding, J. et al. Design and coordination kinematics of an insertable robotic effectors platform for single-port access surgery. IEEE/ASME Trans. Mechatronics 18, 1612-1624 (2013).
[6] ......... Zhao, J., Zheng, X., Zheng, M., Shih, A. J. & Xu, K. An endoscopic continuum testbed for finalizing system characteristics of a surgical robot for NOTES procedures. 2013 IEEE/ASME Int. Conf. Adv. Intell. Mechatronics Mechatronics Hum. Wellbeing, AIM 2013 63-70 (2013). doi:10.1109/AIM.2013.6584069
[7] ......... Burgner, J., Swaney, P. J., Lathrop, R. A., Weaver, K. D. & Webster, R. J. Debulking from within: A robotic steerable cannula for intracerebral hemorrhage evacuation. IEEE Trans. Biomed. Eng. 60, 2567-2575 (2013)
[8] ......... Friedrich, D. T. et al. Teleoperated Tubular Continuum Robots for Transoral Surgery - Feasibility in a Porcine Larynx Model. Int. J. Med. Robot. Comput. Assist. Surg. 1-7 (2018). doi:10.1002/rcs.1928
[9] ......... Langer, M., Amanov, E. & Burgner-Kahrs, J. Stiffening Sheaths for Continuum Robots. Soft Robot. 5, soro.2017.0060 (2018)
[10] ....... Kummer, M. P. et al. Octomag: An electromagnetic system for 5-DOF wireless micromanipulation. IEEE Trans. Robot. 26, 1006-1017 (2010).
[11] ....... Chautems, C. & Nelson, B. J. The tethered magnet: Force and 5-DOF pose control for cardiac ablation. Proc. - IEEE Int. Conf. Robot. Autom. 4837-4842 (2017). doi:10.1109/ICRA.2017.7989562
[12] ....... Greigarn, T. & Cavusoglu, M. C. Task-space motion planning of MRI-actuated catheters for catheter ablation of atrial fibrillation. IEEE Int. Conf. Intell. Robot. Syst. 3476-3482 (2014). doi:10.1109/IROS.2014.6943047
[13] ....... Noonan, D. P., Vitiello, V., Shang, J., Payne, C. J. & Yang, G. Z. A modular, mechatronic joint design for a flexible access platform for MIS. IEEE Int. Conf. Intell. Robot. Syst. 949-954 (2011). doi:10.1109/IROS.2011.6048575
[14]Crews, J. H. & Buckner, G. D. Design optimization of a shape memory alloy-actuated robotic catheter. J. Intell. Mater. Syst. Struct. 23, 545-562 (2012).
[15] ....... Chen, G., Pham, M. T. & Redarce, T. Sensor-based guidance control of a continuum robot for a semi-autonomous colonoscopy. Rob. Auton. Syst. 57, 712-722 (2009).
[16]....... Ikuta, K., Matsuda, Y., Yajima, D. & Ota, Y. Pressure pulse drive: A control method for the precise bending of hydraulic active catheters. IEEE/ASME Trans. Mechatronics 17, 876-883 (2012).
[17] ....... Camacho-Lopez, M., Finkelmann, H., Palffy-Muhoray, P. & Shelley, M. Fast liquid-crystal elastomer swims into the dark. Nat. Mater. 3, 307-310 (2004).
[18] ....... Lagerwall, J. P. F. & Scalia, G. A new era for liquid crystal research: Applications of liquid crystals in soft matter nano-, bio- and microtechnology. Curr. Appl. Phys. 12, 1387-1412 (2012).
[19].......Rogóż, M., Zeng, H., Xuan, C., Wiersma, D. S. & Wasylczyk, P. Light-Driven Soft Robot Mimics Caterpillar Locomotion in Natural Scale. Adv. Opt. Mater. 4, 1689-1694 (2016)
[20]....... Zeng, H. et al. Light-Fueled Microscopic Walkers. Adv. Mater. 27, 3883-3887 (2015)
[21]....... Palagi, S. et al. Structured light enables biomimetic swimming and versatile locomotion of photoresponsive soft microrobots. Nat. Mater. 15, 647-653 (2016)
[22]....... Van Oosten, C. L., Bastiaansen, C. W. M. & Broer, D. J. Printed artificial cilia from liquid-crystal network actuators modularly driven by light. Nat. Mater. 8, 677-682 (2009)
[23]....... Zeng, H., Wani, O. M., Wasylczyk, P., Kaczmarek, R. & Priimagi, A. Self-Regulating Iris Based on Light-Actuated Liquid Crystal Elastomer. Adv. Mater. 29, 1-7 (2017) .
[24]....... Waters, J. T., Li, S., Yao, Y., Lerch, M. M., Aizenberg, M., Aizenberg, J., & Balazs, A. C. (2020). Twist again: Dynamically and reversibly controllable chirality in liquid crystalline elastomer microposts. Science Advances, 6(13). https://doi.org/10.1126/sciadv.aay5349
[25]....... Liu, Y., Wu, W., Wei, J., & Yu, Y. (2017). Visible light responsive liquid crystal polymers containing reactive moieties with good processability. ACS Applied Materials and Interfaces, 9(1), 782-789. https://doi.org/10.1021/acsami.6b11550
[26].......... Yao, Y., Waters, J. T., Shneidman, A. V., Cui, J., Wang, X., Mandsberg, N. K., ... Aizenberg, J. (2018). Multiresponsive polymeric microstructures with encoded predetermined and self-regulated deformability. Proceedings of the National Academy of Sciences of the United States of America, 115(51), 12950-12955. https://doi.org/10.1073/pnas.1811823115
[27]....... Thomsen, D. L., Keller, P., Naciri, J., Pink, R., Jeon, H., Shenoy, D., & Ratna, B. R. (2001). Liquid crystal elastomers with mechanical properties of a muscle. Macromolecules, 34(17), 5868-5875. https://doi.org/10.1021/ma001639q

### Bezugszeichenliste

- A, B, C, D, E, F: Grundstruktur
- Aᵢ, Bᵢ, Cᵢ, Dᵢ, Eᵢ, Fᵢ: Grundstrukturen
- 100: Vorrichtung gemäß Anspruch 1
- 110: integrierter Lichtleiter
- 112: ansteuerbare Lichtquelle
- 114, 116: Element(e) der Grundstruktur A, B, C, D, E oder F
- 118: Gelenkelement der Grundstruktur A, B, C, D, E oder F
- 120: Licht-aktuierbares Material
- 122: Aktuator
- 124: dominante oder nutzbare Wirkrichtung
- 126: Einschnitt
- 128: lokale Materialschwächung
- 130: das zumindest zweite Material im Komposit
- 140: Lichtansteuerung
- 142: Lichtintensität I
- 144: Lichtpolarisation P
- 146: Wellenlänge λ
- 148: Lichtspektrum f(λ)
- 200: Vorrichtung gemäß Anspruch 2
- 300: Vorrichtung gemäß Anspruch 3
- 400: Vorrichtung gemäß Anspruch 4
- 500: Vorrichtung gemäß Anspruch 5
- 600: Vorrichtung gemäß Anspruch 6
- 700: Vorrichtung gemäß Anspruch 7
- 800: Vorrichtung gemäß Anspruch 8
- 900: System gemäß Anspruch 9
- 1000: Verfahren gemäß Anspruch 10

## Patentansprüche

1. Eine Vorrichtung (100), umfassend:
eine Grundstruktur (A, B, C, D, E, F) mit zumindest einem integrierten Lichtleiter (110) und/oder einer ansteuerbaren Lichtquelle (112), wobei diese zwei Elemente (114, 116) oder zwei Elemente (114, 116) und ein Gelenkelement (118), welches zumindest teilweise zwischen den Elementen (114, 116) angeordnet ist, umfasst,
**dadurch gekennzeichnet, dass**
a. zumindest eines der Elemente (114, 116) und/oder das Gelenkelement (118) zumindest teilweise aus zumindest einem Licht-aktuierbaren Material (120) gebildet ist oder zumindest teilweise ein solches aufweist und damit jeweils einen Aktuator (122) mit zumindest einer dominanten oder nutzbaren Wirkrichtung (124) bildet, und
b. der Lichtleiter (110) und/oder die ansteuerbare Lichtquelle (112) derart eingerichtet und in oder an der Grundstruktur (A, B, C, D, E, F) angeordnet sind, dass damit Licht zum oder ans Licht-aktuierbare Material (120) leitbar ist.

2. Vorrichtung (200) gemäß Anspruch 1, wobei zumindest eines der Elemente (114, 116) derart gebildet ist, dass es eine zuvor wählbare Elastizität aufweist.

3. Vorrichtung (300) gemäß Anspruch 1 oder 2, wobei zumindest eine der mindestens einen Wirkrichtung (124) auf zumindest das andere Element (114, 116), eines der anderen Elemente (114, 116), das Gelenkelement (118) oder eines der Gelenkelemente (118) gerichtet ist.

4. Vorrichtung (400) gemäß einem der Ansprüche 1 bis 3,
a. wobei das Licht-aktuierbare Material (120) zumindest ein Material aus der Gruppe von Licht-aktuierbaren Materialien aufweist oder zumindest teilweise daraus gebildet ist, und
b. wobei diese Gruppe Hydrogele und/oder Flüssigkristallpolymere umfasst.

5. Vorrichtung (500) gemäß Anspruch 4,
a. wobei mindestens ein Aktuator (122), derart als Komposit gebildet ist, wobei:
i. dieser Komposit jeweils zumindest zwei verbundene Materialien (120, 130) umfasst, und
ii. zumindest eines der Materialien (120, 130) zumindest teilweise Licht-aktuierbares Material (120) aufweist oder daraus gebildet ist.

6. Vorrichtung (600) gemäß Anspruch 5, wobei:
a. sich die Materialien entsprechend der Wirkrichtung (124) des jeweiligen Licht-aktuierbaren Materials (120) entweder entgegengesetzt zueinander bewegen, oder
b. zumindest eines der Materialien Licht-aktuierbares Material (120) mit einer Wirkrichtung (124) aufweist oder daraus gebildet und zumindest eines der anderen Materialien frei von Licht-aktuierbarem Material ist.

7. Vorrichtung (700) gemäß einem der Ansprüche 1 bis 6, wobei mindestens ein Aktuator (122) zumindest einen Einschnitt (126) oder eine lokale Materialschwächung (128) derart aufweist, dass dadurch im Bereich des Einschnitts (126) oder der lokalen Materialschwächung (128) eine erhöhte elastische Nachgiebigkeit gebildet ist.

8. Vorrichtung (800) gemäß einem der Ansprüche 1 bis 7, wobei mindestens ein Aktuator (122) zumindest eine mit zumindest einem Licht-aktuierbaren Material (120) gefüllten Einschnitt (126) oder eine lokale Materialschwächung (128) derart aufweist, dass damit beim Aktuieren eine Vorzugrichtung implementiert wird.

9. Ein System (900), umfassend zwei Vorrichtungen (100, 200, 300, 400, 500, 600, 700, 800) gemäß einem der Ansprüche 1 bis 8, wobei diese Vorrichtungen (100, 200, 300, 400, 500, 600, 700, 800) miteinander gekoppelt sind.

10. Verfahren (1000) zum Betreiben der Vorrichtung (100, 200, 300, 400, 500, 600, 700, 800) gemäß einem der Ansprüche 1 bis 8 oder des Systems (900) gemäß Anspruch 9, umfassend:
a. Bereitstellen der Vorrichtung (100, 200, 300, 400, 500, 600, 700, 800) gemäß einem der Ansprüche 1 bis 8 oder des Systems (900) gemäß Anspruch 9,
b. Ansteuern zumindest eines Teiles des Licht-aktuierbaren Materials (120) mindestens eines Aktuators (122) mittels Applizieren von Licht über den Lichtleiter (110) und/oder die ansteuerbare Lichtquelle (112) zum jeweiligen Licht-aktuierbaren Material (120) des jeweiligen Aktuators (122) mit einer zuvor wählbaren Intensität (I, 132), Polarisation (P, 134) und/oder Wellenlänge (λ, 136) oder einem Spektrum (f(λ), 138) derart, dass damit die Wirkrichtung (124) des jeweiligen Aktuators (122) gesteuert wird.

11. Verwendung der Vorrichtung (100, 200, 300, 400, 500, 600, 700, 800) gemäß den Ansprüchen 1 bis 8 oder des Systems (900) gemäß Anspruch 9 in der Medizintechnik oder als Kontinuumsroboter.

12. Verwendung des Verfahrens (1000) gemäß Anspruch 10 zum Betreiben der Vorrichtung (100, 200, 300, 400, 500, 600, 700, 800) gemäß einem der Ansprüche 1 bis 8 oder des Systems (900) gemäß Anspruch 9 in der Medizintechnik oder zum Betreiben eines Kontinuumsroboter.
